# EUROPEAN PATENT SPECIFICATION

(11) **EP 1 871 334 B1**
(45) Date of publication and mention of the grant of the patent: **12.10.2011**
(21) Application number: 06739081.5
(22) Date of filing: 21.03.2006
(51) Int. Cl.: A61K 8/44, A61K 8/49, A61K 8/67, A61K 8/97, A61Q 19/02, A61K 36/185, A61K 36/484, A61K 36/258, A61K 36/82, A61K 36/752, A61K 36/42, A61K 36/9062, A61K 36/605, A61K 36/739, A61K 36/53

(54) **SKIN LIGHTENING COMPOSITIONS**
ZUSAMMENSETZUNGEN ZUR HAUTAUFHELLUNG
LOTIONS DE BLANCHIMENT DE LA PEAU

(30) Priority: 23.03.2005 US 664333 P
(43) Date of publication of application: 02.01.2008
(73) Proprietor: Mary Kay, Inc., Dallas, TX 75379-9045 (US)
(72) Inventor: MAJMUDAR, Gopa, Irving, Texas 75061 (US); ZHAO, Wanli, Irving, Texas 75063 (US)
(74) Representative: UEXKÜLL & STOLBERG
(86) International application number: PCT/US2006/010149
(87) International publication number: WO 2006/102289

(56) References cited:
- EP-A- 1 479 374
- WO-A-99/49878
- WO-A-02/062312
- WO-A-03/061768
- WO-A-2005/004830
- WO-A-2005/092283
- WO-A-2006/020164
- US-A- 4 096 240
- US-A- 5 747 006
- US-A1- 2003 180 237
- US-A1- 2004 081 672
- US-A1- 2004 175 347
- US-B1- 6 436 378
- DATABASE WPI Section Ch, Week 200430 Thomson Scientific, London, GB; Class B03, AN 2004-325843 XP002541273 B. G. CHO, J. H. JUNG, S. U. KIM: "Skin whitening cosmetic composition contains ascorbic acid 2-glucoside" & KR 2003 080 869 A (KOREAN COSMETICS CO LTD) 17 October 2003 (2003-10-17)
- DATABASE WPI Section Ch, Week 200468 Thomson Scientific, London, GB; Class B03, AN 2004-693405 XP002541274 N. NAKAGAWA, S. SAKAI, Y. SUMIDA, O. TANNO: "Skin whitening cosmetics, contain nicotinamide, pressed material or extract of plant having antioxidant effect, and skin-whitening component" & JP 2004 262853 A (KANEBO LTD) 24 September 2004 (2004-09-24)
- DATABASE WPI Section Ch, Week 200364 Thomson Scientific, London, GB; Class B04, AN 2003-674111 XP002541275 K. HARA: "Stabilizer for use in cosmetics, quasi-drug of pharmaceutical composition containing oil-soluble licorice extract as skin-whitening agent, contains pyrosulfite and/or sulfite as main component" & JP 2003 113070 A (MOCHIDA PHARM CO LTD) 18 April 2003 (2003-04-18)
- H. S. TALWAR ET AL.: "Differential Regulation of Tyrosinase Activity in Skin of White and Black Individuals In Vivo by Topical Retinoic Acid", THE JOURNAL OF INVESTIGATIVE DERMATOLOGY, vol. 100, no. 6, June 1993 (1993-06), pages 800-805,

## Description

### CROSS-REFERENCE TO RELATED APPLICATIONS

This application claims the benefit of U.S. Provisional Application No. 60/664,333, filed March 23, 2005, the contents of which are incorporated by reference.

### BACKGROUND OF THE INVENTION

### A. Field of the Invention

The present invention relates generally to compositions and methods that can be used in skin-whitening applications. In certain aspects, the compositions of the present invention can include, for example, a combination of ingredients that can be used to whiten skin, even out skin color, or treat hyperpigmentation.

### B. Background of the Invention

The color in human skin is caused by the pigment melanin. Melanin is produced in special dendritic cells, melanocytes, which are found below or between the basal cells of the epidermis of the skin (U.S. Pat. No. 5,411,741). Melanin is synthesized by a reaction cascade triggered by the enzyme tyrosinase (U.S. Pat. No. 5,262,153).

Typical pigmentation is characterized by an even, uniform coloration of the skin. Many individuals have excess melanin pigmentation or a hyperpigmentation patch which can cause pigmentary variation or abnormal pigmentation of the skin. This may lead to unwanted freckles or dark spots such as senile lentigo, liver spots, melasma, brown or age spots, vitiligo, sunburn pigmentation, post-inflammatory hyperpigmentation due to abrasion, bums, wounds or dermatitis, phototoxic reaction and other similar small, fixed pigmented lesions. It is often desirable to lighten these areas or even out the appearance of irregularly pigmented areas of skin. Individuals may also wish to increase fairness or reduce the overall level of pigmentation in the skin. In either case, the hyperpigmentation is usually viewed as cosmetically undesirable and individuals often wish to lighten the skin.

In some instances, the use of one skin lightening ingredient may not be effective for individuals with significant hyperpigmentation, freckles, or age spots, for example. Additionally, previous attempts to combine various skin lightening ingredients have been ineffective, and in some instance, have produced negative results (Talwar 1993).

### SUMMARY OF THE INVENTION

The present invention overcomes deficiencies in the art by providing compositions and methods for their use that can be used to lighten skin. A composition of the present invention includes
(a) ascorbic acid 2-glucoside;
(b) niacinamide;
(c) a combination of cucumber extract and lemon extract;
(d) creatinine; and
(e) undecylenoyl phenylalanine.

Further the compositions of the present invention can include additional ingredients and compounds that are dicussed throughout this document and are incorporated into this section by reference.

In certain aspects, the compositions of the present invention can be formulated into a cosmetic blend. The composition can, in certain embodiments, be chemically compatible. The composition can also include a pH of 1, 2, 3, 4, 5, 6, 7, 8, 9, 10, 11, 12, 13, 14, or more. In certain aspects, the pH of the composition is between about 5 and 8. The compositions can also be included into a cosmetic vehicle. Non limiting examples of cosmetic vehicles include emulsions, creams, lotions, solutions, anhydrous bases, gels, and ointments, and other vehicles that are discussed throughout this document and that are known to those of ordinary skill in the art. In certain aspects, the cosmetic vehicle is an oil-in-water emulsion, a water-in-oil emulsion, an aqueous solution, or hydro-alcoholic solution. The anhydrous base, in non-limiting aspects can be a lipstick or powder. In still other embodiments, the compositions can be included into a cosmetic product. Non-limiting examples of cosmetic products include skin-whitening products, anti-aging products, moisturizing products, foundations, masks, lotions, skin softeners, cleansers, creams (*e.g*., day or night creams), or sunscreens, or other products that are disclosed throughout this document or are known to those of ordinary skill in the art which are incorporated into this section by reference.

In certain embodiments, the composition of the present invention can be adapted or formulated for application at least once, twice, three, four, five, six, seven, eight, nine, ten, eleven, twelve, thirteen, fourteen, fifteen, sixteen, seventeen, or more times a day during use. It is contemplated that the composition of the present invention can be used in a regimen-like format. For example, the regimen can include applying various products to a person's skin in the morning or evening or both. In other aspects, the various products can be applied at various intervals during any time of the day or evening. The intervals can vary depending on the desired effects of a given person or a product. In a non-limiting embodiment, for example, a regimen can include applying a cleanser, a softener, a lotion, a sunscreen, and/or a foundation in the morning; it is contemplated that all, some, or one of these products include a composition of the present invention. Another non-limiting example of a regimen can include applying a cleanser, a mask, a softener, a lotion, and/or a night cream in the evening. It is also contemplated that all, some, or one of these products include a composition of the present invention. In certain aspects, a regimen can include combining morning and evening regimens. It is contemplated that any type of regimen format can be used with the present invention. The regimens can also be varied to the specific needs or desires of a person using the product. The length of time of the regimens can vary. For example, the regimen can by 1, 2, 3, 4, 5, 6, 7, 8, 9, 10, 11, 12, 13, 14, days or more. In other non-limiting examples, the length of time of the regimen can be 3, 4, 5, 6, 7, 8, 9, 10, 15, 20, 25, 30, 35, 40, 45, 50, 60, 70, 80, 90, 100, 200, weeks or longer.

The compositions of the present invention, in non-limiting aspects, can be formulated as a leave-on composition, a rinsing composition, or as a cleansing composition. The compositions can also be formulated to include a sun protection factor (SPF) of 1, 2, 3, 4, 5, 6, 7, 8, 9, 10, 15, 20, 25, 30, 35, 40, 45, 50, or more. The compositions can also, for example, be formulated into a cosmetic product. Non-limiting examples of cosmetic products include skin softeners, day or night lotions, day or night creams, foundations, cleansers, masks, or sunscreens. It is contemplated, however, that the compositions of the present invention can be incorporated into any type of cosmetic product discussed in this document or known to those of ordinary skill in the art.

Compositions according to the invention contain ascorbic acid 2-glucoside.

In certain aspects, the composition comprising cucumber extract and lemon extract can further include sodium citrate. Non-limiting examples of such a composition include a formulation called UNINONTAN U34™.

Compositions according to the invention contain creatinine.

Creatinine has the following formula:

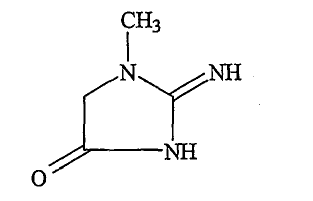

In other non-limiting embodiments of the present invention, the composition can include from about 0.01% to about 5.0% of ascorbic acid 2-glucoside, from about 0.01% to about 5.0% of niacinamide, from about 0.01% to about 5.0% of the extract formulation comprising cucumber extract and lemon extract, from about 0.01% to about 5.0% of creatinine and/or from about 0.01% to about 5.0% of undecylenoyl phenylalanine.

In instances where the composition comprises a second vitamin C derivative, the second vitamin C derivative can be present in an amount of about 0.01% to about 5.0%. The composition of the present invention, in other aspects, may include a licorice extract. In non-limiting embodiments, the licorice extract can be an oil-soluble licorice extract. The licorice can be present in an amount from about 0.01% to about 5.0% of the composition. In other embodiments, the compositions of the present invention can include a botanical blend. In non-limiting examples, the botanical blend can include any one of the following: lemon extract, cucumber extract, green tea extract, ginseng extract, mulberry extract, evening primrose seed extract, thyme extract, galangal extract, burnet extract, or licorice extract. Other ingredients discussed throughout this document and known to those of skill in the art can also be included in the botanical blend. In certain aspects, the botanical blend can be present in the composition in an amount of from about 0.01% to about 5.0% of the composition. As noted throughout this document, the amount of the ingredients and compounds in the composition of the present invention can be similar or different. Additionally, the amounts can vary below, in between, or above the ranges noted above. It is further contemplated that two or more of the ingredients or compounds may be used at concentrations below or above these concentration ranges because of, for example, a synergistic effect between the two or more ingredients. The concentration ranges can also vary to achieve a specific desired result (*e.g*., a person may want to lighten their skin slightly or may want to achieve stronger results).

In other non-limiting embodiments, the compositions of the present invention can include a formulation selected from the group consisting of the formulation described in Table 2, Table 5, Table 7, Table 8, Table 16, and Table 17, below.

In another aspect of the present invention, there is disclosed a method of lightening skin or evening skin tone, treating, preventing, or reducing hyperpigmentation, or reducing the appearance of an age spot, a skin discoloration, or a freckle comprising applying to the skin,

a composition comprising:
(a) ascorbic acid 2-glucoside;
(b) niacinamide;
(c) a combination of cucumber extract and lemon extract;
(d) creatinine; and
(e) undecylenoyl phenylalanine. Additionally, the ingredients in the composition can be formulated into separate compositions, and the separate compositions can be applied to the skin at the same or different times. The composition, in certain aspects, can inhibit, prevent, or reduce melanogenesis in a skin cell, tyrosinase or tyrosinase synthesis in a skin cell, or melanin transport to keratinocytes in a skin cell. In other embodiments, the composition can act as an alpha melanin stimulatory hormone antagonist. In another aspect, the method can be further defined as a method of evening out the pigmentation of the skin. The method can include, in other non-limiting embodiments, applying the composition one, two, three, four, five six, seven, eight, nine, ten, eleven twelve, thirteen, fourteen, fifteen, sixteen, seventeen, or more times a day during use. As noted above, the composition can be used in a regimen-like format. For example, the regimen can include applying various products that have the composition of the present invention to a person's skin in the morning or evening or both. The intervals can vary depending on the desired effects of a given person or product. In a non-limiting embodiment, the method can include, for example, applying a cleanser product, a softener product, a lotion product, a sunscreen product, and/or a foundation product in the morning. The method can also include, in another aspect, applying a cleanser product, a mask product, a softener product, a lotion product, and/or a night cream product in the evening. The skin, in certain non-limiting aspects, can be any skin that is on the human body. Non-limiting examples include facial, head, neck, back, chest, stomach, shoulder, arm, hand, finger, buttock, leg, foot, or toe skin. The method can further be defined, in non-limiting aspects, as reducing the appearance of an age spot, a skin discoloration, or a freckle on the skin. In certain non-limiting aspects, the compositions can include a formulation selected from the group consisting of the formulation described in Table 2,
Table 5, Table 7, Table 8, Table 16, and Table 17, below. In particular methods, the composition includes the formulation described in Table 16. The method can also include applying at least a second, third, fourth, fifth, sixth, seventh, eight, ninth, tenth, eleventh, twelfth, thirteenth, fourteenth, fifteenth, sixteenth, seventeenth, or more skin-lightening composition to the skin. By way of example only, the additional skin lightening composition can include a skin-lightening formulation known to those of ordinary skill in the art and/or those described in this specification. For instance, the additional composition can be selected from the group consisting of the formulation described in Table 2, Table 5, Table 7, Table 8,

Table 16, and Table 17, below. In certain embodiments, the additional composition is the formulation described in Table 17.

It is contemplated that any embodiment discussed in this specification can be implemented with respect to any method or composition of the invention, and *vice versa.* Furthermore, compositions of the invention can be used to achieve methods of the invention.

The terms "inhibiting," "reducing," or "prevention," or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects. For example, "about" can be within 10%, preferably within 5%, more preferably within 1%, and most preferably within 0.5%.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the examples, while indicating specific embodiments of the invention, are given by way of illustration only. Additionally, it is contemplated that changes and modifications within the spirit and scope of the invention will become apparent to those skilled in the art from this detailed description.

### DESCRIPTION OF ILLUSTRATIVE EMBODIMENTS

Many people in the U.S. and world-wide suffer from hyperpigmentation. This can lead to unwanted freckles or dark spots on the skin which can be aesthetically unpleasant. In many instances, it is often desirable to lighten these discolorations or even out the appearance of the irregularly pigmented areas of skin. Also, in certain cultures, people correlate lighter skin tone or color with beauty. Therefore, people in these cultures may feel the need to lighten their natural skin color with skin-lightening agents or compounds.

Previous attempts to lighten skin or even out skin tone have been made. Combing different types of compounds that have skin lightening properties has also been attempted (*e.g.*, PCT/US99/06794, which is incorporated by reference). The present invention is an effective alternative to the skin-whitening compounds and formulas that are currently used to lighten the skin, treat hyperpigmentation, or other skin tone disorders.

The compositions and methods of the present invention can be used, for example, for improving the skin's visual appearance, whitening or lightening the skin's color or tone, treating hyperpigmentation and other related disorders, and evening out a person's skin tone. The compositions of the present invention include a combination of ingredients that can be used to lighten skin.

These and other non-limiting aspects of the present invention are discussed in further detail in the following sections.

### A. Ascorbyl Glucoside

Ascorbyl glucoside is a derivative of ascorbic acid (vitamin C) that includes an attached glucose sugar. The chemical structure of ascorbic acid (Cas. No. 50-81-7) is:

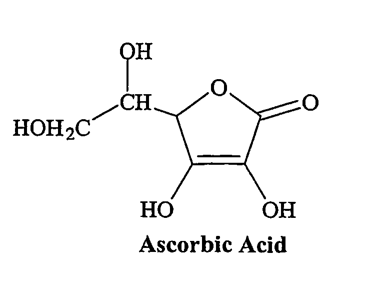

In an ascorbyl glucoside molecule, typically, the glucose is typically attached at an OH group of ascorbic acid. The following is a non-limiting example of one form of ascorbyl glucoside, ascorbic acid-2 glucoside: 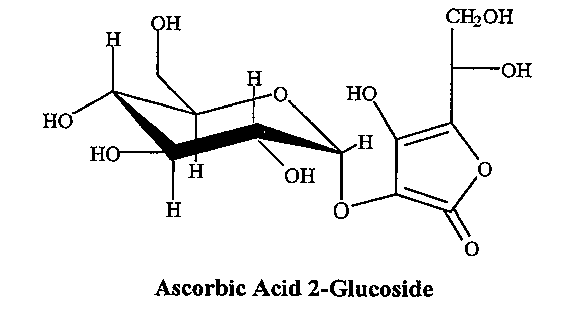 Other non-limiting examples of ascorbyl glucoside include ascorbic acid 1-glucoside (including 1-O-α-D-glucopyranosyl-L-ascorbic acid and 1-O-β-D-glucopyranosyl-L-ascorbic acid), ascorbic acid 2-glucoside (including 2-O-α-D-glucopyranosyl-L-ascorbic acid and 2-O-β-D-glucopyranosyl-L-ascorbic acid), ascorbic acid 3-glucoside (including 3-O-α-D-glucopyranosyl-L-ascorbic acid or 3-O-β-D-glucopyranosyl-L-ascorbic acid), ascorbic acid 5-glucoside (including 5-O-α-D-glucopyranosyl-L-ascorbic acid or 5-O-β-D-glucopyranosyl-L-ascorbic acid), and ascorbic acid 6-glucoside (including 6-O-α-D-glucopyranosyl-L-ascorbic acid or 6-O-β-D-glucopyranosyl-L-ascorbic acid).

Ascorbyl glucoside is commercially available (*e.g.,* Hayashibara Biochemical Laboratories, Inc.). The preparation of ascorbyl glucoside is also known in the art (*e.g.* U.S. Pat. Nos. 5,084,563; 5,252,722; 5,272,136; 5,388,420; 5,432,161; 5,843,907; and 5,508,391).

### B. Niacinamide

Niacinamide (Cas. No. 98-92-0), also known as nicotinamide or pyridine-3-carboxylic acid amide, is a water-soluble amide of nicotinic acid. It is one of the two forms of vitamin B3 and was first isolated from rice bran in 1911 (Niacinamide, Alternative Medicine Review: Volume 7, Number 6, pages 525-529 (2002)). Niacinamide is known to have skin-lightening properties (*e.g*. U.S. Pat. No. 4,096,240; Hakozalci et al, 2002).

The structure of niacinamide includes a pyridine ring that has an amide group at position 3. The molecule formula for niacinamide is C₆H₆N₂O, and its molecular weight is 122.12. The chemical structure of niacinamide is: 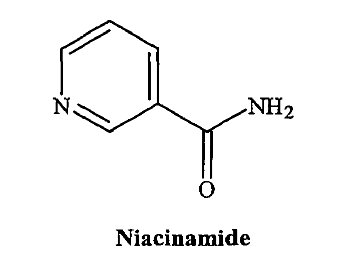 Niacinamide is commercially available (*e.g*., Indian Chemical Industries, Inc.). The preparation of niacinamide is also known in the art.

### C. Extract Formulation

The compositions of the present invention include an extract formulation comprising cucumber extract and lemon extract. The combination of these extracts has skin-lightening properties. In one aspect of this invention, the cucumber and lemon extract combination can be formulated into UNINONTAN U34™.

UNINONTAN-U34^{™} is an effective lightening agent, and it can be combined with the compounds and ingredients that are described in the claims and other sections of this document. By way of example only, vitamin C derivatives, such as MAP and ascorbyl glucoside, can be combined with UNINONTAN U34^{™} without affecting the vitamin C derivatives' stability. A synergistic effect is therefore observed, increasing the total skin lightening effects of the compositions of the present invention.

This allows users of the disclosed compositions to achieve the degree of lightening they wish in a shorter period of time. The compositions also provide the treatments for hyperpigmentation not responsive to traditional treatments. Of course, the present invention may be practiced by combining the raw materials comprising the UNINONTAN-U34^{™} (extract formulation of cucumber extract and lemon extract) product in the amounts specified, or by creating reasonable variations in the ingredients.

The specific ingredients in UNINONTAN U34^{™} include cucumber extract *(cucumis sativus)* (15.0%), lemon extract *(citrus medica limonum)* (16.0%), sodium citrate (20.0%), propylene glycol (23.5%), and water (25.5%). UNINONTAN U34^{™} is sold by Chesham Chemicals, Ltd., located in the United Kingdom.

### D. Creatinine

The compositions of the present invention contain creatinine. Creatinine (or 2-imino-N-methylhydantoin) is a cyclic condensation product which can be obtained by intramolecular elimination of water from creatine. Creatinine has the following structure: 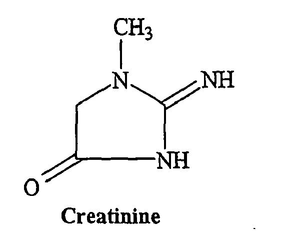 In other aspects of the present invention, creatinine can be formulated into COSMOCAIR C250™. The international company, Degussa, sells COSMOCAIR C250 under its Personal Care Specialties business unit. COSMOCAIR C250 is characterized as a natural amino acid derivative that belongs to the class of guanidino-compounds that can be used in skin brightening products.

### E. Undecylenoyl Phenylalanine

The compositions of the present invention contain undecylenoyl phenylalanine, i.e.

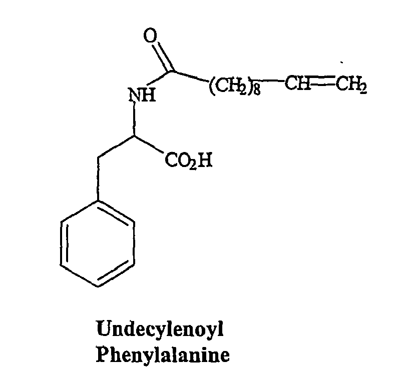

In a preferred and non-limiting embodiment, the above structure can be formulated into SEPIWHITE™ MSH (SEPIWHITET™. SEPIWHITE™ is a formulation that is sold by the French company, Societe D'Exploitation De Produits Pour Les Industries Chemiques (SEPPIC) for use as a skin lightening active ingredient. SEPIWIHTE™ is characterized as an alpha-MSH (melanotropin) antagonist; it reduces the synthesis of melanin pigments effectively while maintaining skin integrity.

### F. Source of Compounds and Extracts

The compounds, extracts, and active ingredients that are described in the claims and specification can be obtained by any means known to a person of ordinary skill in the art. In a non-limiting embodiment, for example, the compounds, extracts, and active ingredients can be isolated by obtaining the source of such compounds and extracts. In many instances, the compounds, extracts, and active ingredients are commercially available. Additionally, the compounds, extracts, and active ingredients can be purified by any number of techniques known to a person of ordinary skill in the art. Non-limiting examples of purification techniques include Polyacrylamide Gel Electrophoresis, High Performance Liquid Chromatography (HPLC), Gel chromatography or Molecular Sieve Chromatography, and Affinity Chromatography.

In other aspects, the compounds, extracts, and active ingredients can be obtained by chemical synthesis or by recombinant means by using conventional techniques. For example, various automatic polypeptide synthesizers and chemical reactions are known and can be used in accordance with known protocols. See, for example, Stewart and Young, (1984); Tam *et al.,* (1983); Merrifield, (1986); and Barany and Merrifield (1979), Houghten (1985).

### G. Modifications and Derivatives

Modifications or derivatives of the chemical structures and compounds disclosed throughout this document are contemplated as being useful with the methods and compositions of the present invention. Derivatives may be prepared and the properties of such derivatives may be assayed for their desired properties by any method known to those of skill in the art.

In certain aspects, "derivative" refers to a chemically modified compound, inhibitor, or stimulator that still retains the desired effects of the prior to the chemical modification. Such derivatives may have the addition, removal, or substitution of one or more chemical moieties on the parent molecule. Non limiting examples of the types modifications that can be made to the compounds and structures disclosed throughout this document include the addition or removal of lower alkanes such as methyl, ethyl, propyl, or substituted lower alkanes such as hydroxymethyl or aminomethyl groups; carboxyl groups and carbonyl groups; hydroxyls; nitro, amino, amide, and azo groups; sulfate, sulfonate, sulfono, sulfhydryl, sulfonyl, sulfoxido, phosphate, phosphono, phosphoryl groups, and halide substituents. Additional modifications can include an addition or a deletion of one or more atoms of the atomic framework, for example, substitution of an ethyl by a propyl; substitution of a phenyl by a larger or smaller aromatic group. Alternatively, in a cyclic or bicyclic structure, hetero atoms such as N, S, or O can be substituted into the structure instead of a carbon atom.

### H. Equivalents

Known and unknown equivalents to the specific compounds, extracts, and active ingredients discussed throughout this document can be used with the compositions and methods of the present invention. The equivalents can be used as substitutes for the specific compounds and extracts. The equivalents can also be used to add to the methods and compositions of the present invention. A person of ordinary skill in the art would be able to recognize and identify acceptable known and unknown equivalents to the specific compounds, extracts, and active ingredients without undue experimentation.

### I. Compositions of the Present Invention

A person of ordinary skill would recognize that the compositions of the present invention can include any number of combinations of compounds and/or extracts, or derivatives therein. It is also contemplated that that the concentrations of the compounds and extracts can vary. In other non-limiting embodiments, for example, the compositions may include in their final form, for example, at least about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.0550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99% or any range derivable therein of at least one of the compounds, extracts, active ingredient, botanical blend or derivatives that are mentioned throughout the specification and claims. In non-limiting aspects, the percentage can be calculated by weight or volume of the total composition. A person of ordinary skill in the art would understand that the concentrations can vary depending on the addition, substitution, and/or subtraction of the compounds, extracts, and substitutes to these compounds and extracts.

The disclosed compositions of the present invention may also include various antioxidants to retard oxidation of one or more components. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

### J. Cosmetic Vehicles

The present compositions are effective in all types of cosmetic vehicles. Non-limiting examples of suitable cosmetic vehicles include emulsions, creams, lotions, solutions (both aqueous and hydro-alcoholic), anhydrous bases (such as lipsticks and powders), gels, and ointments or by other method or any combination of the forgoing as would be known to one of ordinary skill in the art (Remington's, 1990). Variations and other appropriate vehicles will be apparent to the skilled artisan and are appropriate for use in the present invention.

In certain aspects, the cosmetic vehicle is selected from oil-in-water emulsions, hydro-alcoholic solutions, or encapsulated beads in anhydrous systems. With respect to oil-in-water emulsions, such emulsions and their compositions and methods of making are well known in the art. It is important, however, that the concentrations and combinations of the compounds and extracts be selected in such a way that the combinations are chemically compatible and do not form complexes which precipitate from the finished product.

### K. Cosmetic Products

The composition of the present invention can also be used in many cosmetic products including, but not limited to, moisturizing creams, skin benefit creams and lotions, softeners, day lotions, gels, ointments, foundations, night creams, lipsticks, cleansers, toners, masks, or other known cosmetic products or applications. Additionally, the cosmetic products can be formulated as leave-on or rinse-off products. The compositions of the present invention is most preferably used in skin lightening products for the face and other body parts.

### L. Additional Compounds and Agents that Can be Used in Combination With the Present Compositions

Compositions of the present invention can include other beneficial agents and compounds such as, for example, acute or chronic moisturizing agents (including, *e.g.*, humectants, occlusive agents, and agents that affect the natural moisturization mechanisms of the skin), anti-oxidants, sunscreens having UVA and/or UVB protection, emollients, anti-irritants, vitamins, trace metals, anti-microbial agents, botanical extracts, fragrances, and/or dyes and color ingredients.

### 1. Moisturizing Agents

Non-limiting examples of moisturizing agents that can be used with the compositions of the present invention include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrollidone carboxylic acid, potassium PCA, propylene glycol, sodium glucuronate, sodium PCA, sorbitol, sucrose, trehalose, urea, and xylitol.

Other examples include acetylated lanolin, acetylated lanolin alcohol, acrylates/C10-30 alkyl acrylate crosspolymer, acrylates copolymer, alanine, algae extract, aloe barbadensis, aloe-barbadensis extract, aloe barbadensis gel, althea officinalis extract, aluminum starch octenylsuccinate, aluminum stearate, apricot (prunus armeniaca) kernel oil, arginine, arginine aspartate, arnica montana extract, ascorbic acid, ascorbyl palmitate, aspartic acid, avocado (persea gratissima) oil, barium sulfate, barrier sphingolipids, butyl alcohol, beeswax, behenyl alcohol, beta-sitosterol, BHT, birch (betula alba) bark extract, borage (borago officinalis) extract, 2-bromo-2-nitropropane-1,3-diol, butcherbroom (ruscus aculeatus) extract, butylene glycol, calendula officinalis extract, calendula officinalis oil, candelilla (euphorbia cerifera) wax, canola oil, caprylic/capric triglyceride, cardamon (elettaria cardamomum) oil, carnauba (copernicia cerifera) wax, carrageenan (chondrus crispus), carrot (daucus carota sativa) oil, castor (ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palinitate, chamomile (anthemis nobilis) oil, cholesterol, cholesterol esters, cholesteryl hydroxystearate, citric acid, clary (salvia sclarea) oil, cocoa (theobroma cacao) butter, coco-caprylate/caprate, coconut (cocos nucifera) oil, collagen, collagen amino acids, corn (zea mays)oil, fatty acids, decyl oleate, dextrin, diazolidinyl urea, dimethicone copolyol, dimethiconol, dioctyl adipate, dioctyl succinate, dipentaerythrityl hexacaprylate/hexacaprate, DMDM hydantoin, DNA, erythritol, ethoxydiglycol, ethyl linoleate, eucalyptus globulus oil, evening primrose (oenothera biennis) oil, fatty acids, tructose, gelatin, geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (vitis vinifera) seed oil, hazel (corylus americana) nut oil, hazel (corylus avellana) nut oil, hexylene glycol, honey, hyaluronic acid, hybrid safflower (carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, imidazolidinyl urea, iodopropynyl butylcarbamate, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (jasminum officinale) oil, jojoba (buxus chinensis) oil, kelp, kukui (aleurites moluccana) nut oil, lactamide MEA, laneth-16, laneth-10 acetate, lanolin, lanolin acid, lanolin alcohol, lanolin oil, lanolin wax, lavender (lavandula angustifolia) oil, lecithin, lemon (citrus medica limonum) oil, linoleic acid, linolenic acid, macadamia ternifolia nut oil, magnesium stearate, magnesium sulfate, maltitol, matricaria (chamomilla recutita) oil, methyl glucose sesquistearate, methylsilanol PCA, microcrystalline wax, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (olea europaea) oil, orange (citrus aurantium dulcis) oil, palm (elaeis guineensis) oil, palmitic acid, pantethine, panthenol, panthenyl ethyl ether, paraffin, PCA, peach (prunus persica) kernel oil, peanut (arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG40 sorbitan peroleate, PEG-5 soy sterol, PEG-10 soy sterol, PEG-2 stearate, PEG-8 stearate, PEG-20 stearate, PEG-32 stearate, PEG40 stearate, PEG-50 stearate, PEG-100 stearate, PEG-150 stearate, pentadecalactone, peppermint (mentha piperita) oil, petrolatum, phospholipids, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, potassium sorbate, potassium stearate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, quaternium-15, quaternium-18 hectorite, quaternium-22, retinol, retinyl palpitate, rice (oryza sativa) bran oil, RNA, rosemary (rosmarinus officinalis) oil, rose oil, safflower (carthamus tinctorius) oil, sage (salvia officinalis) oil, salicylic acid, sandalwood (santalum album) oil, serine, serum protein, sesame (sesamum indicum) oil, shea butter (butyrospermum parkii), silk powder, sodium chondroitin sulfate, sodium hyaluronate, sodium lactate, sodium palmitate, sodium PCA, sodium polyglutamate, sodium stearate, soluble collagen, sorbic acid, sorbitan laurate, sorbitan oleate, sorbitan palmitate, sorbitan sesquioleate, sorbitan stearate, sorbitol, soybean (glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (helianthus annuus) seed oil, sweet almond (prunus amygdalus dulcis) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (triticum vulgare) germ oil, and ylang ylang (cananga odorata) oil.

### 2. Antioxidants

Non-limiting examples of antioxidants that can be used with the compositions of the present invention include acetyl cysteine, ascorbic acid, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### 3. Compounds Having Ultraviolet Light Absorbing Properties

Non-limiting examples of compounds that have ultraviolet light absorbing properties that can be used with the compounds of the present invention include benzophenone, benzophenone-1, benzophenone-2, benzophenone-3, benzophenone-4 benzophenone-5, benzophenone-6, benzophenone-7, benzophenone-8, benzophenone-9, benzophenone-10, benzophenone-11, benzophenone-12, benzyl salicylate, butyl PABA, cinnamate esters, cinoxate, DEA-methoxycinnamate, diisopropyl methyl cinnamate, ethyl dihydroxypropyl PABA, ethyl diisopropylcinnamate, ethyl methoxycinnamate, ethyl PABA, ethyl urocanate, glyceryl octanoate dimethoxycinnamate, glyceryl PABA, glycol salicylate, homosalate, isoamyl p-methoxycinnamate, PABA, PABA esters, Parsol 1789, and isopropylbenzyl salicylate.

### 4. Structuring Agents

In other non-limiting aspects, the compositions of the present invention can include a structuring agent. Structuring agent, in certain aspects, assist in providing rheological characteristics to the composition to contribute to the composition's stability. In other aspects, structuring agents can also function as an emulsifier or surfactant. Non-limiting examples of structuring agents include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, and mixtures thereof.

### 5. Emulsifiers

In certain preferred aspects of the present invention, the compositions do not include an emulsifier. In other aspects, however, the compositions can include one or more emulsifiers. Emulsifiers can reduce the in interfacial tension between phases and improve the formulation and stability of an emulsion. The emulsifiers can be nonionic, cationic, anionic, and zwitterionic emulsifiers (See McCutcheon's (1986); U.S. Pat. Nos. 5,011,681; 4,421,769; 3,755,560). Non-limiting examples include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, PPG-2 methyl glucose ether distearate, ceteth-10, polysorbate 80, cetyl phosphate, potassium cetyl phosphate, diethanolamine cetyl phosphate, polysorbate 60, glyceryl stearate, PEG-100 stearate, and mixtures thereof.

### 6. Silicone Containing Compounds

In non-limiting aspects, silicone containing compounds include any member of a family of polymeric products whose molecular backbone is made up of alternating silicon and oxygen atoms with side groups attached to the silicon atoms. By varying the -Si-O-chain lengths, side groups, and crosslinking, silicones can be synthesized into a wide variety of materials. They can vary in consistency from liquid to gel to solids.

The silicone containing compounds that can be used in the context of the present invention include those described in this specification or those known to a person of ordinary skill in the art. Non-limiting examples include silicone oils (*e.g*., volatile and nonvolatile oils), gels, and solids. In preferred aspects, the silicon containing compounds includes a silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (e.g., to a particular area such as the skin, hair, or eyes). A "volatile silicone oil" includes a silicone oil have a low heat of vaporization, *i.e*. normally less than about 50 cal per gram of silicone oil. Non-limiting examples of volatile silicone oils include: cyclomethicones such as Dow Corning 344 Fluid, Dow Corning 345 Fluid, Dow Corning 244 Fluid, and Dow Corning 245 Fluid, Volatile Silicon 7207 (Union Carbide Corp., Danbury, Conn.); low viscosity dimethicones, *i.e.* dimethicones having a viscosity of about 50 cst or less (*e.g.,* dimethicones such as Dow Corning 200-0.5 cst Fluid). The Dow Corning Fluids are available from Dow Corning Corporation, Midland, Michigan. Cyclomethicone and dimethicone are described in the Third Edition of the CTFA Cosmetic Ingredient Dictionary (incorporated by reference) as cyclic dimethyl polysiloxane compounds and a mixture of fully methylated linear siloxane polymers end-blocked with trimethylsiloxy units, respectively. Other non-limiting volatile silicone oils that can be used in the context of the present invention include those available from General Electric Co., Silicone Products Div., Waterford, N.Y. and SWS Silicones Div. of Stauffer Chemical Co., Adrian, Michigan.

### 7. Essential Oils

Essential oils include oils derived from herbs, flowers, trees, and other plants. Such oils are typically present as tiny droplets between the plant's cells, and can be extracted by several method know to those of skill in the art (*e.g*., steam distilled, enfleurage (*i.e*., extraction by using fat), maceration, solvent extraction, or mechanical pressing). When these types of oils are exposed to air they tend to evaporate (*i.e*., a volatile oil). As a result, many essential oils are colorless, but with age they can oxidize and become darker. Essential oils are insoluble in water and are soluble in alcohol, ether, fixed oils (vegetal), and other organic solvents. Typical physical characteristics found in essential oils include boiling points that vary from about 160° to 240° C and densities ranging from about 0.759 to about 1.096.

Essential oils typically are named by the plant from which the oil is found. For example, rose oil or peppermint oil are derived from rose or peppermint plants, respectively. Non-limiting examples of essential oils that can be used in the context of the present invention include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang ylang. Other essential oils known to those of skill in the art are also contemplated as being useful within the context of the present invention.

### 8. Thickening Agents

Thickening agents, including thickener or gelling agents, include substances which that can increase the viscosity of a composition. Preferred thickeners includes those that can increase the viscosity of a composition without substantially modifying the efficacy of the active ingredient within the composition. Thickeners can also increase the stability of the compositions of the present invention. In certain aspects of the present invention, preferred thickeners include hydrogenated polyisobutene or trihydroxystearin, or a mixture of both.

Non-limiting examples of additional thickening agents that can be used in the context of the present invention include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol (see U.S. Pat. Nos. 5,087,445; 4,509,949; 2,798,053; CTFA International Cosmetic Ingredient Dictionary, Fourth edition, 1991, pp. 12 and 80). Examples of commercially available carboxylic acid polymers include carbomers, which are homopolymers of acrylic acid crosslinked with allyl ethers of sucrose or pentaerytritol (*e.g*., Carbopol™ 900 series from B. F. Goodrich).

Non-limiting examples of crosslinked polyacrylate polymers include cationic and nonionic polymers. Examples are described in U.S. Pat. Nos. 5,100,660 ; 4,849,484; 4,835,206; 4,628,078; 4,599,379).

Non-limiting examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids.

Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C₁₀ -C₃₀ straight chain or branched chain alkyl group through an ether linkage. Typically these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three unit.

Non-limiting examples of gums that can be used with the present invention include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

### 9. Additional Compounds and Agents

Non-limiting examples of additional compounds and agents that can be used with the compositions of the present invention include additional skin lightening agents (*e.g*. kojic acid, hydroquinone, retinoids and their derivatives) and other known methods of lightening skin, emollients (*e.g*. esters and fatty acids), vitamins (*e.g*. D, E, A, K, and C), trace metals (*e.g*. zinc, calcium and selenium), anti-irritants (*e.g*. steroids and non-steroidal anti-inflammatories), botanical extracts (*e.g*. aloe vera, chamomile, cucumber extract, ginkgo biloba, ginseng, and rosemary), dyes and color ingredients (*e.g*. D&C blue no. 4, D&C green no. 5, D&C orange no. 4, D&C red no. 17, D&C red no. 33, D&C violet no. 2, D&C yellow no. 10, D&C yellow no. 11 and DEA-cetyl phosphate), preservatives (*e.g*. BHA), emollients (*i.e*. organic esters, fatty acids, lanolin and its derivatives, plant and animal oils and fats, and di- and triglycerides), antimicrobial agents (*e.g*., triclosan and ethanol), and fragrances (natural and artificial).

### M. Kits

Kits are also contemplated in certain aspects of the present invention. For example, any of the compositions, compounds, agents, or ingredients described in this specification may be included in a kit. In a non-limiting example, a kit can include a skin whitening composition, a corresponding cosmetic product, or other products and articles of manufacture.

Containers of the kits can include a bottle, dispenser, package, compartment, or other types of containers, into which a component may be placed. The containers can dispense a pre-determined amount of the component (*e.g*. compositions of the present invention). The composition can be dispensed in a spray, an aerosol, or in a liquid form or semi-solid form. The containers can have spray, pump, or squeeze mechanisms. The container can include indicia on its surface. The indicia, for example, can be a word, a phrase, an abbreviation, a picture, or a symbol. The word or phrase can be "Mary Kay," "cosmetic," "sunscreen," *etc*.

Where there is more than one component in the kit (they may be packaged together), the kit also will generally contain a second, third or other additional containers into which the additional components may be separately placed. The kits of the present invention also can include a container housing the components in close confinement for commercial sale. Such containers may include injection or blow-molded plastic containers into which the desired bottles, dispensers, or packages are retained.

A kit can also include instructions for employing the kit components as well the use of any other compositions, compounds, agents, ingredients, or objects not included in the kit. Instructions may include variations that can be implemented. For example, the instructions can include an explanation of how to apply, use, and maintain the products or compositions.

The following examples are included to demonstrate certain non-limiting aspects of the invention. It should be appreciated by those of skill in the art that the techniques disclosed in the examples which follow represent techniques discovered by the inventor to function well in the practice of the invention.

### EXAMPLE 1

### Non-Limiting Examples of Various Types of Skin Whitening Formulations

The following Tables 1-3 provide non-limiting examples of the various types of skin whitening formulations of the present invention that can be comprised in various cosmetic products. As noted throughout this document, it is contemplated that these concentrations ranges can vary. A person of ordinary skill in the art, for example, would recognize that concentration ranges can vary by the addition, removal, or substitution of any one of the listed ingredients. These concentration ranges can also vary depending on the desired effects of any given skin whitening formulation. Further, it should be recognized that the concentration ranges of ingredients can go below or above the concentration ranges noted throughout this document in situations where a synergistic effect is observed between two or more ingredients.

**Table 1: Skin-lightening formulation included in a leave-on skin softener****

| **INGREDIENT** | **% CONCENTRATION** |
|---|---|
| Ascorbyl Glucoside | 0.01 |
| Licorice Extract (oil soluble) | 0.05 |
| Niacinamide | 0.01 |
| MAP | 0.05 |
| UNIMONTAN™ | 0.5 |
| Botanical Blend* | 0.5 |

| | |
|---|---|
| *Botanical blend includes lemon extract, cucumber extract, green tea extract, ginseng extract, mulberry extract, evening primrose seed extract, thyme extract, galangal extract, burnet extract, and licorice extract. **not according to the invention | |

**Table 2: Skin-lightening formulation included in a leave-on day time lotion having an SPF of 15 or a leave-on night cream**

| **INGREDIENT** | **% CONCENTRATION** |
|---|---|
| Ascorbyl Glucoside | 2.0 |
| Licorice Extract (oil soluble) | 0.1 |
| COSMOCAIR™ | 0.2 |
| SEPIWHITE™ | 0.5 |
| Niacinamide | 2.0 |
| MAP | 0.05 |
| UNINONTAN™ | 0.5 |
| Botanical Blend | 0.5 |

| | |
|---|---|
| *Botanical blend includes lemon extract, cucumber extract, green tea extract, ginseng extract, mulberry extract, evening primrose seed extract, thyme extract, galangal extract, burnet extract, and licorice extract. | |

**Table 3: Skin-lightening formulation included in a leave-on foundation, a rinse-off cleanser, and a rinse-off mask****

| **INGREDIENT** | **% CONCENTRATION** |
|---|---|
| Licorice Extract (oil soluble) | 0.1 |
| MAP | 0.05 |
| UNINONTAN™ | 0.5 |
| Botanical Blend | 0.1 |

| | |
|---|---|
| *Botanical blend includes lemon extract, cucumber extract, green tea extract, ginseng extract, mulberry extract, evening primrose seed extract, thyme extract, galangal extract, burnet extract, and licorice extract. ** not according to the invention | |

As disclosed in other sections of this document, derivatives of these ingredients can be used as substitutes. Additionally, other ingredients with similar physiological activities are contemplated as being useful as substitutes or as additional ingredients that can be used with the compositions of the present invention.

### EXAMPLE 2

### Non-Limiting Examples of Preparing Skin Whitening Formulations

This example includes non-limiting procedures of how to make skin softeners, day time lotions, night time lotions, foundations, cleansers, and masks that are described throughout this document. A person of ordinary skill in the art would recognize that these procedures and the ingredients that are used can be varied, removed, added to, or substituted to conform with a specific product or to obtain a desired effect.

**Table 4: Whitening softener*,***

| **Phase** | **Description** | **%** | **Grams** |
|---|---|---|---|
| **A** | Water | 88.72 | 887.20 |
| | TEA | 0.12 | 1.20 |
| | Glycerine | 2.00 | 20.00 |
| | Disodium EDTA | 0.10 | 1.00 |
| | Prodew 400 | 0.01 | 0.10 |
| | Niacinamide | 0.01 | 0.10 |
| | Betafin BP 20 | 1.00 | 10.00 |
| | Net- DG | 0.01 | 0.10 |
| | AA2G | 0.01 | 0.10 |
| | MAP | 0.05 | 0.50 |
| | Uninontan U34 | 0.50 | 5.00 |
| | Actiplex 3535 | 0.01 | 0.10 |
| | Matrixyl 3000 | 0.01 | 0.10 |
| | DMDM Hydantoin | 0.20 | 2.00 |
| | | | |
| **B** | Butylene Glycol | 6.00 | 60.00 |
| | Phenoxyethanol | 0.50 | 5.00 |
| | Methylparaben | 0.20 | 2.00 |
| | PPG-5-Ceteth-20 | 0.50 | 5.00 |
| | Licorice Extract PPC | 0.05 | 0.50 |

| | | | |
|---|---|---|---|
| *Procedure: Add phase A to a beaker in the order presented in the table (*i.e*. from top to bottom). Mix until all of the ingredients are dissolved. Premix phase B in the same manner as phase A. Add phase B to phase A and mix for approximately 20 minutes. These procedures can be performed at room temperature. *not according to the invention | | | |

**Table 5: Whitening day time lotion with sunscreen***

| **Phase** | **Ingredient** | **%** | **Grams** |
|---|---|---|---|
| **A** | Water | 39.15 | 1566.00 |
| | Hydroxyethylcellulose | 0.30 | 12.00 |
| | | | |
| **B** | Propylene Glycol | 3.00 | 120.00 |
| | | | |
| **C** | Phenonip | 1.00 | 40.00 |
| | Betaine | 1.00 | 40.00 |
| | Hispagel Oil/LV | 5.00 | 200.00 |
| | Dimethicone Copolyol PO4 | 0.50 | 20.00 |
| | | | |
| **D** | Sepiwhite | 0.50 | 20.00 |
| | | | |
| **E** | Zinc Oxide/Finsolv TN Disp. | 8.00 | 320.00 |
| | Ethylhexyl P-Methoxycinnamate | 7.50 | 300.00 |
| | Octyl Salicylate | 5.00 | 200.00 |
| | Dimethicone | 0.80 | 32.00 |
| | Oxbenzone | 3.00 | 120.00 |
| | Ceteryl Alcohol/Cetrh 20 | 1.00 | 40.00 |
| | Glyceryl Stearate & PEG 100 | 1.50 | 60.00 |
| | Licorice Extract | 0.05 | 2.00 |
| | Isododecane | 4.00 | 160.00 |
| | | | |
| **F** | Simulgel NS | 2.00 | 80.00 |
| | | | |
| **G** | Glycacil | 0.10 | 4.00 |
| | Proden 400 | 0.10 | 4.00 |
| | Tocopheryl Acetate | 0.10 | 4.00 |
| | Phospholipid EFA | 0.10 | 4.00 |
| | Actiplex 3535 | 0.50 | 20.00 |
| | Matrixyl 3000 | 2.00 | 80.00 |
| | Cosmocare C250 | 0.20 | 8.00 |
| | Niacinamide | 2.00 | 80.00 |
| | Boron Nitride | 0.50 | 20.00 |
| | | | |
| **H** | Water | 2.00 | 80.00 |
| | Citric Acid | 0.95 | 38.00 |
| | | | |
| **I** | Water | 2.00 | 80.00 |
| | Disodium EDTA | 0.10 | 4.00 |
| | Uninontan U34 | 0.50 | 20.00 |
| | MAP | 0.05 | 2.00 |
| | | | |
| **J** | Water | 2.00 | 80.00 |
| | TEA 99% | 0.50 | 20.00 |
| | Ascorbyl Glucoside | 2.00 | 80.00 |
| | | | |
| **K** | Simulgel NS | 1.00 | 40.00 |

| | | | |
|---|---|---|---|
| *Procedure for making this composition. Phase A-Heat water and add Hydroxyethylcellulose. Mix with high speed mixing until batch is clear or transparent and thickened. Add propylene glycol from phase B and continue mixing. Add the ingredients in phase C in order (*i.e*., top to bottom) and mix until uniform. Add the ingredients in phase D and continue mixing batch. Heat the batch to approximately 70-75C°. Obtain, weigh, and begin mixing the ingredients in phase E in a separate beaker and heat to approximately 70-75C°. Add the phase E mixture with the batch when the phase E mixture and the batch are approximately 70-75C°. Continue mixing the batch at approximately 70-75C° with high speed mixing for about 10-15 minutes. Begin cooling the batch. Add the ingredients in phase F to the batch at approximately 60-65C°. The batch should begin to thicken. Continue mixing the batch until it is uniform and then switch to sweep mixing. At approximately 40-45C° add the ingredients in phase G in order with continuous mixing. Premix the phase H ingredients until the citric acid is dissolved in the water. Add the phase H ingredients to the batch at approximately 25-30C°. Premix the phase I ingredients in order and allow each ingredient to dissolve prior to adding the next ingredient. Add the phase I mixture to the batch. Premix the phase J ingredients in order and allow each ingredient to dissolve prior to adding the next ingredient. Add the phase J mixture into the batch. Mix the batch for approximately 10 minutes. Add the phase K ingredients and mix until the batch is uniform, and then mix for approximately 5-10 more minutes. | | | |

**Table 6: Whitening liquid foundation*,***

| **Phase** | **Material** | **Percent** | **Quantity** |
|---|---|---|---|
| **A** | DOW CORNING 9011 | 8.00 | 80.00 |
| | Cyclomethicone | 16.00 | 160.00 |
| | Polyglyceryl-4 Isostear | 1.00 | 10.00 |
| | ACTIPLEX3535 | 0.10 | 1.00 |
| | UNINONTAN | 0.50 | 5.00 |
| | Licorice Root Extract | 0.05 | 0.50 |
| | | | |
| **B** | ANTIQUE IVORY DRY MIX | 20.30 | 203.00 |
| | | | |
| **C** | Water | 49.75 | 497.50 |
| | MATYRIXYL 3000 | 0.10 | 1.00 |
| | MAP | 0.05 | 0.50 |
| | Sodium Chloride | 1.20 | 12.00 |
| | | | |
| **D** | Propylene Glycol | 1.95 | 19.50 |
| | GERMABEN 2 | 1.00 | 10.00 |

| | | | |
|---|---|---|---|
| *Procedure: Mix the ingredients in phase A until uniform to creat a batch. Add the ingredients in phase B to the batch. Homogenize the batch with phase B until uniform. Combine the phase C ingredients into a separate beaker and mix until the mixture is uniform. Premix the phase D ingredients and add to the phase C mixture. Add the phase C and D mixture into the batch in a slow progression . Homogenize the batch for approximately 30 minutes. *not according to the invention | | | |

**Table 7: Whitening essence lotion formulation***

| **Phase** | **Description** | **%** | **Grams** |
|---|---|---|---|
| **A** | Water | 44.80 | 224.00 |
| | TEA | 1.70 | 8.50 |
| | Sepiwhite MSH | 0.50 | 2.50 |
| | Propylene Glycol | 6.00 | 30.00 |
| | Glycerine | 2.00 | 10.00 |
| | Disodium EDTA | 0.10 | 0.50 |
| | Prodew 400 | 0.10 | 0.50 |
| | Uninontan U34 | 0.50 | 2.50 |
| | Cosmocair C 250 | 0.20 | 1.00 |
| | Net- DG | 0.10 | 0.50 |
| | AA2G | 2.00 | 10.00 |
| | Niacinamide | 2.00 | 10.00 |
| | Betafin BP 20 | 1.00 | 5.00 |
| | Actiplex 3535 | 0.50 | 2.50 |
| | Matrixyl 3000 | 2.00 | 10.00 |
| | | | |
| **B** | Carbopol 940 2% | 15.00 | 75.00 |
| | | | |
| **C** | Water | 5.00 | 25.00 |
| | Timiron Super Blue | 1.00 | 5.00 |
| | | | |
| **D** | Permethyl 99A | 4.50 | 22.50 |
| | Dimethicone | 3.50 | 17.50 |
| | Silicone HL-88 | 1.00 | 5.00 |
| | Vitamin E Acetate | 0.10 | 0.50 |
| | Licorice Ext. | 0.05 | 0.25 |
| | DC 193 Fluid | 0.60 | 3.00 |
| | Boron Nitride | 0.50 | 2.50 |
| | Phenonip | 1.00 | 5.00 |
| | | | |
| **E** | Water | 2.00 | 10.00 |
| | MAP | 0.05 | 0.25 |
| | | | |
| **F** | Sepigel 305 | 2.20 | 11.00 |

| | | | |
|---|---|---|---|
| *Procedure: Combine the phase A ingredients and mix until the batch is uniform. Add the phase B ingredients into the batch. Mix the batch and homomix for approximately 2 minutes. Premix the phase C ingredients and add to the batch by mixing. Add the phase G ingredients into the batch by mixing. Premix the phase E ingredients and add to the batch by mixing. Premix the phase F ingredients and add to the batch by mixing. | | | |

**Table 8: Whitening night cream***

| **Phase** | **Description** | **%** | **Grams** |
|---|---|---|---|
| **A** | Water | 38.90 | 194.50 |
| | Glycerin | 3.00 | 15.00 |
| | Betafm BP-20 | 1.00 | 5.00 |
| | Disodium EDTA | 0.10 | 0.50 |
| | Net DG | 0.10 | 0.50 |
| | Sepiwhite MSH | 0.50 | 2.50 |
| | Carbopol 940 2% | 15.00 | 75.00 |
| | | | |
| **B** | Polysynlane | 1.00 | 5.00 |
| | Isostearyl Neopentanoate | 5.00 | 25.00 |
| | Dimethicone | 1.00 | 5.00 |
| | Arlacel 165 | 3.40 | 17.00 |
| | Cetyl Alcohol | 1.00 | 5.00 |
| | Shea Butter | 0.60 | 3.00 |
| | Isostearic Acid | 3.00 | 15.00 |
| | | | |
| **C** | TEA | 1.50 | 7.50 |
| | TiO2 Oil Dispersion | 0.20 | 1.00 |
| | Boron Nitride | 1.00 | 5.00 |
| | | | |
| **D** | Cyclomethicone | 6.00 | 30.00 |
| | Silicone HL-88 | 1.00 | 5.00 |
| | DC 193 Fluid | 0.50 | 2.50 |
| | | | |
| **E** | Water | 6.00 | 30.00 |
| | TEA | 1.00 | 5.00 |
| | AA2G | 2.00 | 10.00 |
| | Cosmocair C250 | 0.20 | 1.00 |
| | Niacinamide | 2.00 | 10.00 |
| | MAP | 0.05 | 0.25 |
| | | | |
| **F** | Glycasil L | 0.10 | 0.50 |
| | Licorice Ext. | 0.05 | 0.25 |
| | Prodew 400 | 0.10 | 0.50 |
| | Tocopheryl Acetate | 0.10 | 0.50 |
| | Uninontan U34 | 0.50 | 2.50 |
| | Actiplex 3535 | 0.50 | 2.50 |
| | Matrixyl 3000 | 2.00 | 10.00 |
| | Phenonip | 1.00 | 5.00 |
| | Sepigel 305 | 0.60 | 3.00 |

| | | | |
|---|---|---|---|
| *Procedure: Combine the phase A ingredients and heat to approximately 75°C. Combine and heat the phase B ingredients in a separate beaker to approximately 75°C. Combine the phase A and B mixtures to form a batch. Combine the phase C ingredients and add to the batch by mixing for approximately 20 minutes. Allow the batch to cool to approximately 65°C and then add the phase D ingredients and mix for approximately 20 minutes. Allow the batch to cool to approximately 45°C and then add the phase E ingredients. Mix and add the phase F ingredient to the batch. Homomix the batch for approximately 5 minutes and allow the batch to coll to approximately 28-30°C. | | | |

**Table 9: Whitening cleanser*, not according to the invention**

| **Phase** | **Description** | **%** |
|---|---|---|
| **A** | Water | 44.800 |
| | Butylene Glycol | 0.500 |
| | TiO2 | 0.500 |
| | | |
| **B** | Sodium Cocoyl Isethionate | 17.000 |
| | | |
| **C** | Polysorbate 60K | 0.500 |
| | Tauronol WS Conc | 5.000 |
| | EDTA | 0.300 |
| | Cetyl Alcohol | 1.900 |
| | Cocamidopropyl Betaine | 4.000 |
| | NaCl | 0.250 |
| | | |
| **D** | Water | 3.000 |
| | TEA 99% | 0.020 |
| | SF-1 Carbpopol | 2.850 |
| | | |
| **E** | Water | 2.850 |
| | TEA 99% | 2.780 |
| | | |
| **F** | Stearic Acid | 10.000 |
| | | |
| **G** | Germaben II | 0.950 |
| | | |
| **H** | Oil Soluble Licorice Extract | 0.050 |
| | Actiplex 3535 | 0.100 |
| | Matrixyl 3000 | 0.100 |
| | Lavender Extract | 1.000 |
| | | |
| **I** | Water | 1.050 |
| | Uninontan | 0.500 |

| | | |
|---|---|---|
| *Procedure: Premix the phase I ingredients in a weight boat and let stand at room temperature in a separate beaker. Mix the phase A ingredients and heat to approximately 85°C. Add the phase B ingredients with the phase A ingredients to form a batch and allow the batch to melt. Maintain the batch at approximately 80-85°C. Add the phase C ingredients one at a time into the batch by mixing. Premix the phase D ingredients one at a time and in order (*i.e*. top to bottom). Allow the batch to cool to approximately 65-70°C. Add to the phase D ingredients to the batch. Premix the phase E ingredients and add to the batch by mixing. Add the phase E ingredients to the batch by mixing. Allow the batch to cool to approximately 60-65°C. Add the phase F ingredients and change to sweep mixing. Maintain the batch at approximately 60°C until the batch is melted. Check batch to make sure that all of the stearic acid has melted and that the batch is smooth and uniform. Allow the batch to cool to 40-45°C by using low sweeps. Add the phase G ingredients to the batch by mixing. Add the phase H ingredients into the batch by mixing. Add the phase I ingredients into the batch by mixing. Allow the batch to cool to approximately 25-30°C. * not according to the invention | | |

**Table 10: Whitening mask*, not according to the invention**

| **Phase** | **Description** | **%** | **G** |
|---|---|---|---|
| **A** | Water | 55.700 | 278.500 |
| | Veegum | 1.000 | 5.000 |
| | | | |
| **B** | Glycerin | 8.000 | 40.000 |
| | Disodium EDTA | 0.100 | 0.500 |
| | Kaolin | 5.000 | 25.000 |
| | TiO2 | 7.000 | 35.000 |
| | Bentonite | 2.000 | 10.000 |
| | | | |
| **C** | Stearic Acid | 2.000 | 10.000 |
| | Sorbitan Stearate | 2.000 | 10.000 |
| | Arlacel 165 | 6.000 | 30.000 |
| | Candelilla Wax | 0.500 | 2.500 |
| | Dimethicone | 1.000 | 5.000 |
| | Cetyl Alcohol | 1.000 | 5.000 |
| | Stearyl Alcohol | 0.500 | 2.500 |
| | Polysynlane | 3.000 | 15.000 |
| | | | |
| **D** | TEA | 0.600 | 3.000 |
| | | | |
| **E** | Polyethylene | 0.800 | 4.000 |
| | Phenonip | 1.000 | 5.000 |
| | Actiplex 3535 | 0.100 | 0.500 |
| | Uninontan | 0.500 | 2.500 |
| | Licorice Extract | 0.050 | 0.250 |
| | Matrixyl 3000 | 0.100 | 0.500 |
| | | | |
| **F** | Water | 2.000 | 10.000 |
| | MAP | 0.050 | 0.250 |
| | | | |
| | | 100.000 | 500.000 |

| | | | |
|---|---|---|---|
| *Preparation: Add the ingredients in phase A to a beaker and mix for approximately 20 minutes to create a batch. Add the phase B ingredients into the batch and mix until the batch is uniform. Heat the batch to approximately 75°C. Add the phase C ingredients into a separate beaker and heat to approximately 75°C. Mix the phase C ingredients into the batch. Add the phase D ingredients into the batch and mix for approximately 20 minutes. Begin cooling the batch to approximately 45°C. Add the phase E ingredients into the batch. Premix the phase F ingredients and then add to the batch. Continue cooling the batch to room temperature (approximately 20-25°C). * not according to the invention | | | |

### EXAMPLE 3

### Efficacy of the Essence Skin Lotion

The effectiveness of the essence skin lotion composition that is described in Table 7 was tested on twenty subjects/panelists. Table 11 shows the results of this self assessment study.

**Table 11***

| | **28 Days** | **56 Days** |
|---|---|---|
| Overall skin improvement | 50% | 64% |
| Overall product performance | 60% | 69% |
| The product provides natural skin whitening/lightening | 59% | 73% |
| The product provides an even skin tone | 59% | 77% |
| The product lightens existing dark spots and freckles | 55% | 63% |

| | | |
|---|---|---|
| *The panelists included 20 females with an Asian background. The whitening essence lotion was applied to the forearm twice a day. After 4 and 8 weeks of product use, the panelists rated their skin condition on a 5-point scale as compared to the condition at the start of the study. The scale ranged from the assessed parameter being much less improved, somewhat less improved, no change, somewhat greater improved, and much greater improved. The values represent the percent of panelists who perceived improvement at the given point in time. | | |

### EXAMPLE 4

### Efficacy of Skin Whitening Regimen

Additional studies were performed on female panelists in the United States and in Thailand. These studies included all of the compositions described in Tables 4-10. The study parameters included applying various combinations of these compositions to the panelist's skin in a regime-like format in the morning and evening. For example, the panelists used the following compositions in the morning and in the following order: (i) the cleanser (Table 9); (ii) the softener (Table 4); (iii) the essence lotion (Table 7); (iv) the day lotion (Table 5); and (v) the foundation (Table 6). The foundation included three different shades or colors (*e.g*.., foundation ivory 105, foundation ivory soft, and foundation antique ivory) of which the subjects selected one. The evening regimen included applying the following compositions in order: (i) the cleanser (Table 9); (ii) the mask (table 10); (iii) the softener formulation (Table 4); (iv) the essence lotion (Table 7); and (v) the night cream (Table 8).

The effectiveness of this regimen was tested on fifty female volunteers in the U.S. (the U.S. Study) and on 41 females volunteers in Thailand (the Thailand Study). The U.S. Study included both objective testing (Table 12) and subjective testing (Table 13). The Thailand Study included objective testing only (Table 14). The following tables provide data showing the synergistic effects of the combination of ingredients.

**Table 12 (U.S. Objective Study)**

| **Benefit** | **% Improvement Compared to Baseline** | | | |
|---|---|---|---|---|
| | **2 weeks** | **4 weeks** | **6 weeks** | **8 weeks** |
| Skin Moisture | 28.70 | 40.30 | 54.10 | 63.60 |
| Reduction in freckles and age spots | 12.40 | 16.60 | 21.30 | 28.40 |
| Dryness | 42.00 | 56.50 | 63.20 | 70.70 |
| Skin Firmness | 21.80 | 30.70 | 36.70 | 41.40 |
| Skin softness/Suppleness | 21.30 | 34.50 | 43.60 | 54.20 |
| Lines & Wrinkles | 28.20 | 39.40 | 49.40 | 56.40 |
| Surface fine lines | 29.30 | 42.30 | 51.20 | 60.30 |
| Skin Smoothness | 21.10 | 28.50 | 33.30 | 39.60 |
| Clarity | 8.30 | 12.80 | 15.10 | 18.20 |
| Surface Contour | 19.40 | 27.50 | 38.30 | 47.30 |
| Skin Tone | 14.20 | 21.50 | 30.00 | 36.20 |

The data in Table 12 were obtained by using objective methods that included instrumental measurements and/or expert grading systems. The results were obtained at 2, 4, 6, and 8 weeks during the regimen use by the subjects. Skin moisture/hydration was measured using impedance measurements with the Nova Dermal Phase Meter. The impedance meter measures changes in skin moisture content. The outer layer of the skin has distinct electrical properties. When skin is dry it conducts electricity very poorly. As it becomes more hydrated increasing conductivity results. Consequently, changes in skin impedance (related to conductivity) can be used to assess changes in skin hydration. In the present study, the unit was calibrated according to instrument instructions for each testing day. A notation of temperature and relative humidity was made. Subjects were evaluated as follows : prior to measurement they will equilibrate in a room with defined humidity (30-50%) and temperature (68-72C). Three separate impedance reading were made on each side of the face, recorded and averaged. The T5 setting was used on the impedance meter that averages the impedance values of every five seconds application to the face. Changes were reported with statistical variance and significance.

Skin clarity and the reduction in freckles and age spots was evaluated using a Minolta Chromometer. Changes in skin color were assessed to determine irritation potential due to product treatment using the a* values of the Minolta Chroma Meter. The a* value measures changes in skin color in the red region. This is used to determine whether the product is inducing irritation. The measurements were made on each side of the face and averaged, as left and right facial values. Skin clarity can also be measured using the Minolta Meter. The measurement is a combination of the a*, b, and L values of the Minolta Meter and is related to skin brightness, and correlates well with skin smoothness and hydration. Skin reading is taken as above. Skin clarity is defined as L/C where C is chroma and is defined as (a²+ b²)^{1/2}.

Skin dryness, surface fine lines, skin smoothness, and skin tone were evaluated with clinical grading techniques. For example, clinical grading of skin dryness was determined by a five point standard Kligman Scale: (0) skin is soft and moist; (1) skin appears normal with no visible dryness; (2) skin feels slightly dry to the touch with no visible flaking; (3) skin feels dry, tough, and has a whitish appearance with some scaling; and (4) skin feels very dry, rough, and has a whitish appearance with scaling. Evaluations were made independently by two clinicians and averaged.

Clinical grading of skin tone was performed via a ten point analog numerical scale: (10) even skin of uniform, pinkish brown color. No dark, erythremic, or scaly patches upon examination with a hand held magnifying lens. Microtexture of the skin very uniform upon touch; (7) even skin tone observed without magnification. No scaly areas, but slight discolorations either due to pigmentation or erythema. No discolorations more than 1 cm in diameter; (4) both skin discoloration and uneven texture easily noticeable. Slight scaliness. Skin rough to the touch in some areas; and (1) uneven skin coloration and texture. Numerous areas of scaliness and discoloration, either hypopigmented, erythremic or dark spots. Large areas of uneven color more than 1 cm in diameter. Evaluations were made independently by two clinicians and averaged.

Clinical grading of skin smoothness was analyzed via a ten point analog numerical scale: (10) smooth, skin is moist and glistening, no resistance upon dragging finger across surface; (7) somewhat smooth, slight resistance; (4) rough, visibly altered, friction upon rubbing; and (1) rough, flaky, uneven surface. Evaluations were made independently by two clinicians and averaged. Skin smoothness and wrinkle reduction can be assessed visually by using the methods disclosed in Packman *et al.* (1978). For example, at each subject visit, the depth, shallowness and the total number of superficial facial lines (SFLs) of each subject can be carefully scored and recorded. A numerical score was obtained by multiplying a number factor times a depth/width/length factor. Scores are obtained for the eye area and mouth area (left and right sides) and added together as the total wrinkle score.

Skin firmness was measured using a Hargens ballistometer, a device that evaluates the elasticity and firmness of the skin by dropping a small body onto the skin and recording its first two rebound peaks. The ballistometry is a small lightweight probe with a relatively blunt tip (4 square mm-contact area) was used. The probe penetrates slightly into the skin and results in measurements that are dependent upon the properties of the outer layers of the skin, including the stratum corneum and outer epidermis and some of the dermal layers.

Skin softness/suppleness was evaluated using the Gas Bearing Electrodynamometer, an instrument that measures the stress/strain properties of the skin. The viscoelastic properties of skin correlate with skin moisturization. Measurements were obtained on the predetermined site on the cheek area by attaching the probe to the skin surface with double-stick tape. A force of approximately 3.5 gm is applied parallel to the skin surface and the skin displacement is accurately measured. Skin suppleness is then calculated and is expressed as DSR (Dynamic Spring Rate in gm/mm).

The appearance of lines and wrinkles on the skin was evaluated using replicas, which is the impression of the skin's surface. Silicone rubber like material is used. Replica is analyzed by image analysis. In conjunction with the clinical assessment of SFL's, described above, changes in the visibility of lines and wrinkles were objectively quantified via the taking of silicon replicas form the subjects' face and analyzing the replicas image using a computer image analysis system. Replicas were taken from the eye area and the neck area, and photographed with a digital camera using a low angle incidence lighting. The digital images were analyzed with an image processing program and the are of the replicas covered by wrinldes or fine lines was determined.

The surface contour of the skin was measured by using the profilometer/Stylus method. This includes either shining a light or dragging a stylus across the replica surface. The vertical displacement of the stylus is fed into a computer via a distance transducer, and after scanning a fixed length of replica a cross-sectional analysis of skin profile is generated as a two-dimensional curve. This scan can be repeated any number of times along a fix axis to generate a simulated 3-D picture of the skin. Ten random sections of the replicas using the stylus technique were obtained and combined to generate average values. The values of interest include Ra which is the arithmetic mean of all roughness (height) values computed by integrating the profile height relative to the mean profile height. Rt which is the maximum vertical distance between the highest peak and lowest trough, and Rz which is the mean peak amplitude minus the mean peak height. Values are given as a calibrated value in mm. Equipment is standardized prior to each use by scanning metal standards of know values. Ra Value is computed by the following equation: Rₐ = Standardize roughness; *l*ₘ = the traverse (scan) length; and |y| = the absolute value of the location of the profile relative to the mean profile height (x-axis).

In other non-limiting aspects, the efficacy of the compositions of the present invention can be evaluated by using a skin analog, such as, for example, MELANODERM™. Melanocytes, one of the cells in the skin analog, stain positively when exposed to L-dihydroxyphenyl alanine (L-DOPA), a precursor of melanin. The skin analog, MELANODERM™, can be treated with a variety of bases containing the compositions and whitening agents of the present invention or with the base alone as a control. Alternatively, an untreated sample of the skin analog can be used as a control.

**Table 13 (U.S. Subjective Study)**

| | **2 Weeks** | | **4 Weeks** | | **6 Weeks** | | **8 Weeks** | |
|---|---|---|---|---|---|---|---|---|
| | **No of Panelists** | **%** | **No of Panelists** | **%** | **No of Panelists** | **%** | **No of Panelists** | **%** |
| **Skin looks naturally Whiter/Lighter** | | | | | | | | |
| Agree strongly | 18 | 36.00 | 29 | 58.00 | 35 | 70.00 | 40 | 80.00 |
| Agree somewhat | 20 | 40.00 | 15 | 30.00 | 12 | 24.00 | 8 | 16.40 |
| Neither agree nor disagree | 10 | 20.00 | 6 | 12.00 | 3 | 6.00 | 2 | 4.00 |
| Disagree somewhat | 2 | 4.00 | 0 | 0.00 | 0 | 0.00 | 0 | 0.00 |
| Disagree strongly | 0 | 0.00 | 0 | 0.00 | 0 | 0.00 | 0 | 0.00 |
| Sum of first two boxes | **38.00** | **76.00** | **44.00** | **88.00** | **47.00** | **94.00** | **48.00** | **96.00** |
| **Skin appears more luminous and fair** | | | | | | | | |
| Agree strongly | 16 | 32.00 | 26 | 52.00 | 32 | 64.00 | 37 | 74.00 |
| Agree somewhat | 19 | 38.00 | 14 | 28.00 | 11 | 22.00 | 9 | 18.00 |
| Neither agree nor disagree | 9 | 18.00 | 6 | 12.00 | 4 | 8.00 | 2 | 4.00 |
| Disagree somewhat | 6 | 12.00 | 4 | 8.00 | 3 | 6.00 | 2 | 4.00 |
| Disagree strongly | 0 | 0.00 | 0 | 0.00 | 0 | 0.00 | 0 | 0.00 |
| Sum of first two boxes | **35.00** | **70.00** | **40.00** | **80.00** | **43.00** | **86.00** | **46.00** | **92.00** |
| **Skin tone looks more even** | | | | | | | | |
| Agree strongly | 17 | 34.00 | 24 | 48.00 | 31 | 62.00 | 35 | 70.00 |
| Agree somewhat | 18 | 36.00 | 16 | 32.00 | 13 | 26.00 | 10 | 20.00 |
| Neither agree nor disagree | 10 | 20.00 | 8 | 16.00 | 5 | 10.00 | 4 | 8.00 |
| Disagree somewhat | 5 | 10.00 | 2 | 4.00 | 1 | 2.00 | 1 | 2.00 |
| Disagree strongly | 0 | 0.00 | 0 | 0.00 | 0 | 0.00 | 0 | 0.00 |
| Sum of first two boxes | **35.00** | **70.00** | **40.00** | **80.00** | **44.00** | **88.00** | **45.00** | **90.00** |
| **Existing dark spots and freckles look lighter** | | | | | | | | |
| Agree strongly | 15 | 30.00 | 21 | 42.00 | 28 | 56.00 | 32 | 64.00 |
| Agree somewhat | 18 | 36.00 | 18 | 36.00 | 15 | 30.00 | 15 | 30.00 |
| Neither agree nor disagree | 12 | 24.00 | 8 | 16.00 | 5 | 10.00 | 3 | 6.00 |
| Disagree somewhat | 4 | 8.00 | 3 | 6.00 | 2 | 4.00 | 0 | 0.00 |
| Disagree strongly | 1 | 2.00 | 0 | 0.00 | 0 | 0.00 | 0 | 0.00 |
| Sum of first two boxes | **33.00** | **66.00** | **39.00** | **78.00** | **43.00** | **86.00** | **47.00** | **94.00** |
| **Improvement in skin's imperfection** | | | | | | | | |
| Agree strongly | 8 | 16.00 | 14 | 28.00 | 23 | 46.00 | 29 | 58.00 |
| Agree somewhat | 11 | 22.00 | 13 | 26.00 | 17 | 34.00 | 16 | 32.00 |
| Neither agree nor disagree | 16 | 32.00 | 15 | 30.00 | 8 | 16.00 | 5 | 10.00 |
| Disagree somewhat | 10 | 20.00 | 5 | 10.00 | 2 | 4.00 | 0 | 0.00 |
| Disagree strongly | 5 | 10.00 | 3 | 6.00 | 0 | 0.00 | 0 | 0.00 |
| Sum of first two boxes | **19.00** | **38.00** | **27.00** | **54.00** | **40.00** | **80.00** | **45.00** | **90.00** |
| **Skin looks more radiant** | | | | | | | | |
| Agree strongly | 15 | 30.00 | 25 | 50.00 | 30 | 60.00 | 36 | 72.00 |
| Agree somewhat | 20 | 40.00 | 15 | 30.00 | 15 | 30.00 | 11 | 22.00 |
| Neither agree nor disagree | 10 | 20.00 | 7 | 14.00 | 4 | 8.00 | 3 | 6.00 |
| Disagree somewhat | 5 | 10.00 | 3 | 6.00 | 1 | 2.00 | 0 | 0.00 |
| Disagree strongly | 0 | 0.00 | 0 | 0.00 | 0 | 0.00 | 0 | 0.00 |
| Sum of first two boxes | **35.00** | **70.00** | **40.00** | **80.00** | **45.00** | **90.00** | **47.00** | **94.00** |
| **Moisturizes the skin** | | | | | | | | |
| Agree strongly | 20 | 40.00 | 30 | 60.00 | 36 | 72.00 | 42 | 84.00 |
| Agree somewhat | 22 | 44.00 | 16 | 32.00 | 14 | 28.00 | 8 | 16.00 |
| Neither agree nor disagree | 6 | 12.00 | 4 | 8.00 | 0 | 0.00 | 0 | 0.00 |
| Disagree somewhat | 2 | 4.00 | 0 | 0.00 | 0 | 0.00 | 0 | 0.00 |
| Disagree strongly | 0 | 0.00 | 0 | 0.00 | 0 | 0.00 | 0 | 0.00 |
| Sum of first two boxes | **42.00** | **84.00** | **46.00** | **92.00** | **50.00** | **100.00** | **50.00** | **100.00** |
| **Leaves skin smooth and supple** | | | | | | | | |
| Agree strongly | 19 | 38.00 | 28 | 56.00 | 33 | 66.00 | 40 | 80.00 |
| Agree somewhat | 24 | 48.00 | 17 | 34.00 | 15 | 30.00 | 9 | 18.00 |
| Neither agree nor disagree | 4 | 8.00 | 5 | 10.00 | 2 | 4.00 | 1 | 2.00 |
| Disagree somewhat | 3 | 6.00 | 0 | 0.00 | 0 | 0.00 | 0 | 0.00 |
| Disagree strongly | 0 | 0.00 | 0 | 0.00 | 0 | 0.00 | 0 | 0.00 |
| Sum of first two boxes | **43.00** | **86.00** | **45.00** | **90.00** | **48.00** | **96.00** | **49.00** | **98.00** |
| **Skin looks and feels healthier** | | | | | | | | |
| Agree strongly | 12 | 24.00 | 19 | 38.00 | 24 | 48.00 | 29 | 58.00 |
| Agree somewhat | 14 | 28.00 | 20 | 40.00 | 19 | 38.00 | 18 | 36.00 |
| Neither agree nor disagree | 12 | 24.00 | 7 | 14.00 | 6 | 12.00 | 3 | 6.00 |
| Disagree somewhat | 10 | 20.00 | 3 | 6.00 | 1 | 2.00 | 0 | 0.00 |
| Disagree strongly | 2 | 4.00 | 1 | 2.00 | 0 | 0.00 | 0 | 0.00 |
| Sum of first two boxes | **26.00** | **52.00** | **39.00** | **78.00** | **43.00** | **86.00** | **47.00** | **94.00** |
| **Skin looks younger** | | | | | | | | |
| Agree strongly | 10 | 20.00 | 18 | 36.00 | 26 | 52.00 | 31 | 62.00 |
| Agree somewhat | 15 | 30.00 | 17 | 34.00 | 16 | 32.00 | 16 | 32.00 |
| Neither agree nor disagree | 13 | 26.00 | 9 | 18.00 | 5 | 10.00 | 2 | 4.00 |
| Disagree somewhat | 9 | 18.00 | 5 | 10.00 | 3 | 6.00 | 1 | 2.00 |
| Disagree strongly | 3 | 6.00 | 1 | 2.00 | 0 | 0.00 | 0 | 0.00 |
| Sum of first two boxes | **25.00** | **50.00** | **35.00** | **70.00** | **42.00** | **84.00** | **47.00** | **94.00** |
| **Skin improved overall** | | | | | | | | |
| Agree strongly | 20 | 40.00 | 34 | 68.00 | 41 | 82.00 | 47 | 94.00 |
| Agree somewhat | 28 | 56.00 | 16 | 32.00 | 9 | 18.00 | 3 | 6.00 |
| Neither agree nor disagree | 2 | 4.00 | 0 | 0.00 | 0 | 0.00 | 0 | 0.00 |
| Disagree somewhat | 0 | 0.00 | 0 | 0.00 | 0 | 0.00 | 0 | 0.00 |
| Disagree strongly | 0 | 0.00 | 0 | 0.00 | 0 | 0.00 | 0 | 0.00 |
| Sum of first two boxes | **48.00** | **96.00** | **50.00** | **100.00** | **50.00** | **100.00** | **50.00** | **100.00** |
| **This regimen is better than the one you usually use?** | | | | | | | | |
| Agree strongly | 12 | 24.00 | 20 | 40.00 | 24 | 48.00 | 30 | 60.00 |
| Agree somewhat | 14 | 28.00 | 16 | 32.00 | 18 | 36.00 | 15 | 30.00 |
| Neither agree nor disagree | 16 | 32.00 | 10 | 20.00 | 6 | 12.00 | 4 | 8.00 |
| Disagree somewhat | 5 | 10.00 | 3 | 6.00 | 2 | 4.00 | 1 | 2.00 |
| Disagree strongly | 3 | 6.00 | 1 | 2.00 | 0 | 0.00 | 0 | 0.00 |
| Sum of first two boxes | **26.00** | **52.00** | **36.00** | **72.00** | **42.00** | **84.00** | **45.00** | **90.00** |

**Table 14 (Thailand Subjective Study)**

| | **2 Weeks** | | **4 Weeks** | | **6 Weeks** | | **8 Weeks** | |
|---|---|---|---|---|---|---|---|---|
| | **No of Panelist** | **%** | **No of Panelist** | **%** | **No of Panelist** | | **No of Panelist** | **%** |
| **Skin looks naturally Whiter/Lighter** | | | | | | | | |
| Agree strongly | 5 | 12.20 | 8 | 19.51 | 11 | 26.83 | 13 | 31.71 |
| Agree somewhat | 25 | 60.98 | 31 | 75.61 | 29 | 70.73 | 28 | 68.29 |
| Neither agree nor disagree | 11 | 26.83 | 2 | 4.88 | 1 | 2.44 | 0 | 0.00 |
| Disagree somewhat | 0 | 0.00 | 0 | 0.00 | 0 | 0.00 | 0 | 0.00 |
| Disagree strongly | 0 | 0.00 | 0 | 0 | 0 | 0 | 0 | 0 |
| Sum of first two boxes | **30.00** | **73.17** | **39.00** | **95.12** | **40.00** | **97.56** | **41.00** | **100.00** |
| **Skin appears more luminous and fair** | | | | | | | | |
| Agree strongly | 6 | 14.63 | 8 | 19.51 | 13 | 31.71 | 14 | 34.15 |
| Agree somewhat | 24 | 58.54 | 28 | 68.29 | 26 | 63.41 | 26 | 63.41 |
| Neither agree nor disagree | 10 | 24.39 | 5 | 12.20 | 2 | 4.88 | 1 | 2.44 |
| Disagree somewhat | 1 | 2.44 | 0 | 0.00 | 0 | 0.00 | 0 | 0.00 |
| Disagree strongly | 0 | 0.00 | 0 | 0 | 0 | 0 | 0 | 0 |
| Sum of first two boxes | **30.00** | **73.17** | **36.00** | **87.80** | **39.00** | **95.12** | **40.00** | **97.56** |
| **Skin tone looks more even** | | | | | | | | |
| Agree strongly | 6 | 14.63 | 7 | 17.07 | 9 | 21.95 | 10 | 24.39 |
| Agree somewhat | 22 | 53.66 | 30 | 73.17 | 29 | 70.73 | 28 | 68.29 |
| Neither agree nor disagree | 11 | 26.83 | 4 | 9.76 | 3 | 7.32 | 3 | 7.32 |
| Disagree somewhat | 2 | 4.88 | 0 | 0.00 | 0 | 0.00 | 0 | 0.00 |
| Disagree strongly | 0 | 0.00 | 0 | 0 | 0 | 0 | 0 | 0 |
| Sum of first two boxes | **28.00** | **68.29** | **37.00** | **90.24** | **38.00** | **92.68** | **38.00** | **92.68** |
| **Existing dark spots and freckles look lighter** | | | | | | | | |
| Agree strongly | 4 | 9.76 | 8 | 19.51 | 11 | 26.83 | 12 | 29.27 |
| Agree somewhat | 22 | 53.66 | 24 | 58.54 | 25 | 60.98 | 27 | 65.85 |
| Neither agree nor disagree | 14 | 34.15 | 9 | 21.95 | 5 | 12.20 | 2 | 4.88 |
| Disagree somewhat | 1 | 2.44 | 0 | 0.00 | 0 | 0.00 | 0 | 0.00 |
| Disagree strongly | 0 | 0.00 | 0 | 0 | 0 | 0 | 0 | 0 |
| Sum of first two boxes | **26.00** | **63.41** | **32.00** | **78.05** | **36.00** | **87.80** | **39.00** | **95.12** |
| **Improvement in skin's imperfection** | | | | | | | | |
| Agree strongly | 9 | 21.95 | 11 | 26.83 | 10 | 24.39 | 13 | 31.71 |
| Agree somewhat | 24 | 58.54 | 26 | 63.41 | 28 | 68.29 | 25 | 60.98 |
| Neither agree nor disagree | 7 | 17.07 | 3 | 7.32 | 3 | 7.32 | 3 | 7.32 |
| Disagree somewhat | 1 | 2.44 | 1 | 2.44 | 0 | 0.00 | 0 | 0.00 |
| Disagree strongly | 0 | 0.00 | 0 | 0 | 0 | 0 | 0 | 0 |
| Sum of first two boxes | **33.00** | **80.49** | **37.00** | **90.24** | **38.00** | **92.68** | **38.00** | **92.68** |
| **Skin looks more radiant** | | | | | | | | |
| Agree strongly | 6 | 14.63 | 8 | 19.51 | 15 | 36.59 | 14 | 34.15 |
| Agree somewhat | 27 | 65.85 | 27 | 65.85 | 26 | 63.41 | 27 | 65.85 |
| Neither agree nor disagree | 7 | 17.07 | 5 | 12.20 | 0 | 0.00 | 0 | 0.00 |
| Disagree somewhat | 0 | 0.00 | 1 | 2.44 | 0 | 0.00 | 0 | 0.00 |
| Disagree strongly | 1 | 2.44 | 0 | 0 | 0 | 0 | 0 | 0 |
| Sum of first two boxes | **33.00** | **80.49** | **35.00** | **85.37** | **41.00** | **100.00** | **41.00** | **100.00** |
| | | | | | | | | |
| **Moisturizes the skin** | | | | | | | | |
| Agree strongly | 8 | 19.51 | 10 | 24.39 | 12 | 29.27 | 13 | 31.71 |
| Agree somewhat | 21 | 51.22 | 27 | 65.85 | 26 | 63.41 | 26 | 63.41 |
| Neither agree nor disagree | 12 | 29.27 | 4 | 9.76 | 3 | 7.32 | 2 | 4.88 |
| Disagree somewhat | 0 | 0.00 | 0 | 0.00 | 0 | 0.00 | 0 | 0.00 |
| Disagree strongly | 0 | 0.00 | 0 | 0 | 0 | 0 | 0 | 0 |
| Sum of first two boxes | **29.00** | **70.73** | **37.00** | **90.24** | **38.00** | **92.68** | **39.00** | **95.12** |
| **Leaves skin smooth and supple** | | | | | | | | |
| Agree strongly | 11 | 26.83 | 12 | 29.27 | 14 | 34.15 | 15 | 36.59 |
| Agree somewhat | 24 | 58.54 | 26 | 63.41 | 24 | 58.54 | 23 | 56.10 |
| Neither agree nor disagree | 6 | 14.63 | 3 | 7.32 | 3 | 7.32 | 3 | 7.32 |
| Disagree somewhat | 0 | 0.00 | 0 | 0.00 | 0 | 0.00 | 0 | 0.00 |
| Disagree strongly | 0 | 0.00 | 0 | 0 | 0 | 0 | 0 | 0 |
| Sum of first two boxes | **35.00** | **85.37** | **38.00** | **92.68** | **38.00** | **92.68** | **38.00** | **92.68** |
| **Skin looks and feels healthier** | | | | | | | | |
| Agree strongly | 9 | 21.95 | 9 | 21.95 | 12 | 29.27 | 15 | 36.59 |
| Agree somewhat | 27 | 65.85 | 28 | 68.29 | 26 | 63.41 | 23 | 56.10 |
| Neither agree nor disagree | 3 | 7.32 | 3 | 7.32 | 3 | 7.32 | 3 | 7.32 |
| Disagree somewhat | 1 | 2.44 | 1 | 2.44 | 0 | 0.00 | 0 | 0.00 |
| Disagree strongly | 1 | 2.44 | 0 | 0 | 0 | 0 | 0 | 0 |
| Sum of first two boxes | **36.00** | **87.80** | **37.00** | **90.24** | **38.00** | **92.68** | **38.00** | **92.68** |
| **Skin looks younger** | | | | | | | | |
| Agree strongly | 2 | 4.88 | 6 | 14.63 | 4 | 9.76 | 8 | 19.51 |
| Agree somewhat | 19 | 46.34 | 24 | 58.54 | 29 | 70.73 | 28 | 68.29 |
| Neither agree nor disagree | 11 | 26.83 | 10 | 24.39 | 8 | 19.51 | 5 | 12.20 |
| Disagree somewhat | 8 | 19.51 | 1 | 2.44 | 0 | 0.00 | 0 | 0.00 |
| Disagree strongly | 1 | 2.44 | 0 | 0 | 0 | 0 | 0 | 0 |
| Sum of first two boxes | **21.00** | **51.22** | **30.00** | **73.17** | **33.00** | **80.49** | **36.00** | **87.80** |
| **Skin improved overall** | | | | | | | | |
| Agree strongly | 9 | 21.95 | 12 | 29.27 | 16 | 39.02 | 19 | 46.34 |
| Agree somewhat | 28 | 68.29 | 27 | 65.85 | 23 | 56.10 | 20 | 48.78 |
| Neither agree nor disagree | 3 | 7.32 | 2 | 4.88 | 2 | 4.88 | 2 | 4.88 |
| Disagree somewhat | 1 | 2.44 | 0 | 0.00 | 0 | 0.00 | 0 | 0.00 |
| Disagree strongly | 0 | 0.00 | 0 | 0 | 0 | 0 | 0 | 0 |
| Sum of first two boxes | **37.00** | **90.24** | **39.00** | **95.12** | **39.00** | **95.12** | **39.00** | **95.12** |
| **This regimen is better than the one you usually use?** | | | | | | | | |
| Agree strongly | 8 | 19.51 | 9 | 21.95 | 11 | 26.83 | 12 | 29.27 |
| Agree somewhat | 27 | 65.85 | 24 | 58.54 | 25 | 60.98 | 27 | 65.85 |
| Neither agree nor disagree | 5 | 12.20 | 6 | 14.63 | 5 | 12.20 | 2 | 4.88 |
| Disagree somewhat | 1 | 2.44 | 2 | 4.88 | 0 | 0.00 | 0 | 0.00 |
| Disagree strongly | 0 | 0.00 | 0 | 0 | 0 | 0 | 0 | 0 |
| Sum of first two boxes | **35.00** | **85.37** | **33.00** | **80.49** | **36.00** | **87.80** | **39.00** | **95.12** |

The following Table 15 provides a summary of the subjective data that was obtained in the U.S. and Thailand Studies (Tables 13 and 14, respectively).

**Table 15**

| **% Panelists perceived improvements after 8 weeks** | | |
|---|---|---|
| | **Thailand Study** | **U.S. Study** |
| Skin looks naturally Whiter/Lighter | 100 | 96 |
| Skin appears more luminous and fair 98 92 | 98 | 92 |
| Skin tone looks more even | 93 | 90 |
| Existing dark spots and freckles look lighter | 95 | 94 |
| Improves skin's imperfection | 93 | 90 |
| Skin looks more radiant | 100 | 94 |
| Moisturizes the skin | 95 | 100 |
| Leaves the skin smooth and supple | 93 | 98 |
| Skin looks and feels healthier | 93 | 94 |
| Skin looks younger | 89 | 94 |
| Skin improved overall | 95 | 100 |
| This regimen is better than the one you usually use | 95 | 90 |

### EXAMPLE 5

### Non-Limiting Examples of TimeWise Essence and TimeWise Night Cream-Skin Whitening Formulations

The following Tables 16-17 provide non-limiting examples of TimeWise Essence and TimeWise Night Cream Formulations. As discussed above, these concentrations ranges can vary.

**Table 16: TimeWise Essence skin-lightening formulation***

| **Phase** | **Description** | **%** | **Grams** |
|---|---|---|---|
| **A** | Water | 44.85 | 448.50 |
| | TEA | 1.70 | 17.00 |
| | Sepiwhite MSH | 0.50 | 5.00 |
| | Propylene Glycol | 6.00 | 60.00 |
| | Glycerine | 2.00 | 20.00 |
| | Disodium EDTA | 0.10 | 1.00 |
| | Prodew 400 | 0.10 | 1.00 |
| | Uninontan U34 | 0.50 | 5.00 |
| | Cosmocair C 250 | 0.20 | 2.00 |
| | Net- DG | 0.10 | 1.00 |
| | AA2G | 2.00 | 20.00 |
| | Niacinamide | 2.00 | 20.00 |
| | Betafin BP 20 | 1.00 | 10.00 |
| | Actiplex 3691 | 0.50 | 5.00 |
| | Matrixyl 3000 | 2.00 | 20.00 |
| | | | |
| **B** | Carbopol 940 2% | 15.00 | 150.00 |
| | | | |
| **C** | Water | 5.00 | 50.00 |
| | Timiron Super Blue | 1.00 | 10.00 |
| | | | |
| **D** | Permethyl 99A | 4.50 | 45.00 |
| | Dimethicone | 3.50 | 35.00 |
| | Silicone HL-88 | 1.00 | 10.00 |
| | Vitamin E Acetate | 0.10 | 1.00 |
| | DC 193 Fluid | 0.60 | 6.00 |
| | Boron Nitride | 0.50 | 5.00 |
| | Phenonip | 1.00 | 10.00 |
| | | | |
| **E** | Water | 2.00 | 20.00 |
| | MAP | 0.05 | 0.50 |
| | | | |
| **F** | Sepigel 305 | 2.20 | 22.00 |

| | | | |
|---|---|---|---|
| *Procedure: Combine phase A ingredients and mix until uniform. Add phase B ingredients into the batch. Mix batch and homomix for approximately 2 minutes. Premix the phase C ingredients and add to the batch by mixing. Add the phase D ingredients into the batch by mixing. Premix the phase E ingredients and add to the batch by mixing. Add phase F ingredients and mix for approximately 30 minutes. | | | |

**Table 17: TimeWise Night Cream skin-lightening formulation***

| **Phase** | **Description** | **%** | **Grams** |
|---|---|---|---|
| **A** | Water | 38.95 | 194.75 |
| | Glycerin | 3.00 | 15.00 |
| | Betafin BP-20 | 1.00 | 5.00 |
| | Disodium EDTA | 0.10 | 0.50 |
| | Net DG | 0.10 | 0.50 |
| | Sepiwhite MSH | 0.50 | 2.50 |
| | Carbopol 940 2% | 15.00 | 75.00 |
| | | | |
| **B** | Polysynlane | 1.00 | 5.00 |
| | Isostearyl Neopentanoate | 5.00 | 25.00 |
| | Dimethicone | 1.00 | 5.00 |
| | Arlacel 165 | 3.40 | 17.00 |
| | Cetyl Alcohol | 1.00 | 5.00 |
| | Shea Butter | 0.60 | 3.00 |
| | Isostearic Acid | 3.00 | 15.00 |
| | | | |
| **C** | TEA | 1.50 | 7.50 |
| | TiO₂ Oil Dispersion | 0.20 | 1.00 |
| | Boron Nitride | 1.00 | 5.00 |
| | | | |
| **D** | Cyclomethicone | 6.00 | 30.00 |
| | Silicone HL-88 | 1.00 | 5.00 |
| | DC 193 Fluid | 0.50 | 2.50 |
| | | | |
| **E** | Water | 6.00 | 30.00 |
| | TEA | 1.00 | 5.00 |
| | AA2G | 2.00 | 10.00 |
| | Cosmocair C250 | 0.20 | 1.00 |
| | Niacinamide | 2.00 | 10.00 |
| | MAP | 0.05 | 0.25 |
| | | | |
| **F** | Glycasil L | 0.10 | 0.50 |
| | Prodew 400 | 0.10 | 0.50 |
| | Tocopheryl Acetate | 0.10 | 0.50 |
| | Uninontan U34 | 0.50 | 2.50 |
| | Actiplex 3535 | 0.50 | 2.50 |
| | Matrixyl 3000 | 2.00 | 10.00 |
| | Phenonip | 1.00 | 5.00 |
| | Sepigel 305 | 0.60 | 3.00 |

| | | | |
|---|---|---|---|
| *Procedure: Combine the phase A ingredients and heat to approximately 75°C. Combine and heat the phase B ingredients in a separate beaker to approximately 75°C. Combine the phase A and B mixtures to form a batch. Combine the phase C ingredients and add to the batch by mixing for approximately 20 minutes. Allow the batch to cool to approximately 65°C and then add the phase D ingredients and mix for approximately 20 minutes. At 45°C premix phase E and add to batch. Mix until uniform. Add the phase F ingredient to batch. Mix until uniform. Homomix batch for approximately 5 minutes and allow the batch to cool to approximately 28-30°C. | | | |

### EXAMPLE 6

### Efficacy of TimeWise Essence skin-lightening formulation

**Study Design:** The TimeWise Essence formulation described in Table 16 was subjected to a twelve week clinical efficacy study conducted at KGL Skin Study Center, PA. Visual assessments for facial attributes were performed by a Dermatologist and evaluated at baseline and weeks 2, 4, 8 and 12. Color photographs of each panelist were taken at baseline and week 12.

**Product Application:** Subjects were asked to apply the TimeWise Essence formulation in the morning and evening daily for 12 weeks. If subjects were exposed to the sun for more than 30 minutes during the day, they were required to use an SPF 15 sunscreen lotion.

**Panelist Accountability:** 44 out of 49 subjects completed the study. Three subjects were dropped from the study for non-compliance and the other subject withdrew for personal reasons. There was no adverse event reported for this study.

### Dermatologist Assessments:

**Table 18: Mean Percent Change from Baseline**

| **Facial Attributes** | **Week 2 n=46** | **Week 4 n=45** | **Week 8 n=45** | **Week 12 n=44** |
|---|---|---|---|---|
| **Texture** | 24% | 29% | 34% | 36% |
| **Clarity** | 17% | 26% | 31% | 34% |
| **Even Skin Tone** | 15% | 19% | 26% | 29% |
| **Discrete Pigment** (age or brown spots, freckles) | 4% | 13% | 22% | 31% |
| **Mottled Pigment** (large sun spots) | 4% | 17% | 28% | 39% |
| **Fine Wrinkling** | 12% | 18% | 21% | 25% |
| **Coarse Wrinkling** | NS | NS | NS | NS |
| **Sallowness** (yellowed skin tone) | 13% | 20% | 24% | 27% |
| **Laxity** | NS | NS | 3% | 14% |
| **Undereye Puffmess** | NS | NS | NS | NS |
| **Sagging** | 2% | 4% | 4% | 4% |
| **Turgor** | 9% | 9% | 16% | 23% |
| **Overall Photodamage** | 7% | 16% | 22% | 25% |

| | | | | |
|---|---|---|---|---|
| NS: Not significantly different from baseline | | | | |

**Table 19: Percent of Panelists Showed Improvement from Baseline**

| **Facial Attributes** | **Week 2 n=46** | **Week 4 n=45** | **Week 8 n=45** | **Week 12 n=44** |
|---|---|---|---|---|
| **Texture** | 83% | 91% | 100% | 100% |
| **Clarity** | 63% | 89% | 98% | 100% |
| **Even Skin Tone** | 59% | 71% | 82% | 84% |
| **Discrete Pigment** | 13% | 44% | 64% | 82% |
| **Mottled Pigment** | 11 % | 47% | 60% | 68% |
| **Fine Wrinkling** | 44% | 64% | 76% | 84% |
| **Coarse Wrinkling** | NS | NS | NS | NS |
| **Sallowness** | 50% | 64% | 76% | 75% |
| **Laxity** | NS | NS | 13% | 50% |
| **Undereye Puffiness** | NS | NS | NS | NS |
| **Sagging** | 9% | 13% | 13% | 16% |
| **Turgor** | 28% | 33% | 60% | 80% |
| **Overall Photodamge** | 33% | 69% | 89% | 98% |

| | | | | |
|---|---|---|---|---|
| NS: Not significantly different from baseline | | | | |

### EXAMPLE 7

### Efficacy of TimeWise Essence and Night Cream formulations

**Study Design:** The TimeWise Essence and Night Cream formulations described in Tables 16 and 17, respectively, were subjected to a twelve week clinical efficacy study conducted at KGL Skin Study Center, PA. Visual assessments for facial attributes were performed by a Dermatologist and evaluated at baseline and weeks 2, 4, 8 and 12. Color photographs of each panelist were taken at baseline and week 12.

**Product Application:** Subjects were asked to apply the Essence formulation in the morning and evening and the Night Cream formulation in the evening daily for 12 weeks. If subjects were exposed to the sun for more than 30 minutes during the day, they were required to use an SPF 15 sunscreen lotion.

**Panelist Accountability:** 46 out of 49 subjects completed the study. Three subjects withdrew from the study for personal reasons. There was no adverse event reported for this study.

### Dermatologist Assessments:

**Table 20: Mean Percent Change from Baseline (Magnitude of change)**

| **Facial Attributes** | **Week 2 n=47** | **Week 4 n=47** | **Week 8 n=47** | **Week 12 n=46** |
|---|---|---|---|---|
| **Texture** (smoothness) | 35% | 42% | 44% | 42% |
| **Clarity** | 24% | 33% | 37% | 38% |
| **Even Skin Tone** | 20% | 28% | 30% | 34% |
| **Discrete Pigment** (age or brown spots, freckles) | 7% | 17% | 25% | 30% |
| **Mottled Pigment** (sun spots) | 9% | 21% | 34% | 39% |
| **Fine Wrinkling** | 18% | 24% | 26% | 26% |
| **Coarse Wrinkling** | NS | 7% | 8% | 12% |
| **Sallowness** (yellowed skin tone) | 24% | 29% | 28% | 32% |
| **Laxity** (firmness) | NS | NS | NS | 8% |
| **Undereye Puffmess** | NS | 4% | 5% | 7% |
| **Sagging** | NS | NS | NS | 3% |
| **Turgor** | 4% | 11% | 14% | 15% |
| **Overall Photodamage** | 13% | 21% | 23% | 25% |

| | | | | |
|---|---|---|---|---|
| NS: Not significantly different from baseline | | | | |

**Table 21: Percent of Panelists Showed Improvement from Baseline**

| **Facial Attributes** | **Week 2 n=47** | **Week 4 n=47** | **Week 8 n=47** | **Week 12 n=46** |
|---|---|---|---|---|
| **Texture** | 94% | 98% | 98% | 100% |
| **Clarity** | 83% | 98% | 100% | 100% |
| **Even Skin Tone** | 75% | 89% | 92% | 96% |
| **Discrete Pigment** | 28% | 57% | 75% | 80% |
| **Mottled Pigment** | 26% | 49% | 60% | 67% |
| **Fine Wrinkling** | 64% | 83% | 85% | 87% |
| **Coarse Wrinkling** | NS | 19% | 21% | 33% |
| **Sallowness** | 68% | 77% | 72% | 80% |
| **Laxity** | NS | NS | NS | 33% |
| **Undereye Puffiness** | NS | 15% | 21% | 28% |
| **Sagging** | NS | NS | NS | 11 % |
| **Turgor** | 15% | 43% | 53% | 57% |
| **Overall Photodamge** | 64% | 85% | 92% | 94% |

| | | | | |
|---|---|---|---|---|
| NS: Not significantly different from baseline | | | | |

All of the compositions and/or methods disclosed and claimed in this specification can be made and executed without undue experimentation in light of the present disclosure.

More specifically, it will be apparent that certain agents which are both chemically and physiologically related may be substituted for the agents described herein while the same or similar results would be achieved.

### REFERENCES

The following references provide exemplary procedural or other details supplementary to those set forth herein.
U.S. Patent 4,096,240
U.S. Patent 5,084,563
U.S. Patent 5,252,722
U.S. Patent 5,262,153
U.S. Patent 5,272,136
U.S. Patent 5,388,420
U.S. Patent 5,411,741
U.S. Patent 5,432,161
U.S. Patent 5,508,391
U.S. Patent 5,843,907
U.S. Patent 6,235,910
U.S. Patent 6,235,910
U.S. Pub. 2003/0180237
U.S. Pub. 2003/0180237

Alternative Medicine Review, 7(6):525-529, 2002. Barany and Merrifield, In: The Peptides, Gross and Meienhofer (Eds.), Academic Press, NY, 1-284, 1979.
Hakozaki et al., Br. J. Dermatol., 147:20-31, 2002.
Houghten et al., Infect. Immun., 48(3):735-740., 1985.
Merrifield, Science, 232(4748):341-347, 1986.
Packman and Gams, J. Soc. Cos. Chem., 29:70-90, 1978.
PCT Appln. PCT/US99/06794
PCT Appln. WO 03/061768
PCT Appln. WO 03011241
PCT Appln. WO 03011242
PCT Appln. WO 04064801
Remington's Pharmaceutical Sciences, 18th Ed. Mack Printing Company, 1289-1329, 1990.
Stewart and Young, In: Solid Phase Peptide Synthesis, 2d. ed., Pierce Chemical Co., 1984.
Talwar et al., J. Invest Dermatol., 100:800-805, 1993.
Tam et al., J. Am. Chem. Soc., 105:6442, 1983.

## Claims

1. A skin-lightening composition comprising:
(a) ascorbic acid 2-glucoside;
(b) niacinamide;
(c) a combination of cucumber extract and lemon extract;
(d) creatinine; and
(e) undecylenoyl phenylalanine.

2. The skin lightening composition of claim 1, wherein the composition comprises:
(a) from 0,01 to 5% by weight of the composition of ascorbic acid 2-glucoside;
(b) from 0,01 to 5% by weight of the composition of niacinamide;
(c) from 0,01 to 5% by weight of the composition of a combination of cucumber extract and lemon extract;
(d) from 0,01 to 5% by weight of the composition of creatinine; and
(e) from 0,01 to 5% by weight of the composition of undecylenoyl phenylalanine.

3. The skin lightening composition of claim 1, wherein the composition has the formulation
described in the following table
| **Phase** | **Description** | **%** | **Grams** |
|---|---|---|---|
| **A** | Water | 44.80 | 224.00 |
| | TEA (triethanolamine) | 1.70 | 8.50 |
| | Sepiwhite MSH (undecylenoyl phenylalanine) | 0.50 | 2.50 |
| | Propylene Glycol | 6.00 | 30.00 |
| | Glycerine | 2.00 | 10.00 |
| | Disodium EDTA | 0.10 | 0.50 |
| | Prodew 400 | 0.10 | 0.50 |
| | Uninontan U34 (a combination of cucumber extract and lemon extract | 0.50 | 2.50 |
| | Cosmocair C 250 (creatinine) | 0.20 | 1.00 |
| | Net- DG | 0.10 | 0.50 |
| | AA2G (ascorbic acid 2-glucoside) | 2.00 | 10.00 |
| | Niacinamide | 2.00 | 10.00 |
| | Betafin BP 20 | 1.00 | 5.00 |
| | Actiplex 3535 | 0.50 | 2.50 |
| | Matrixyl 3000 | 2.00 | 10.00 |
| | | | |
| **B** | Carbopol 940 2% | 15.00 | 75.00 |
| | | | |
| **C** | Water | 5.00 | 25.00 |
| | Timiron Super Blue | 1.00 | 5.00 |
| | | | |
| **D** | Permethyl 99A | 4.50 | 22.50 |
| | Dimethicone | 3.50 | 17.50 |
| | Silicone HL-88 | 1.00 | 5.00 |
| | Vitamin E Acetate | 0.10 | 0.50 |
| | Licorice Ext. | 0.05 | 0.25 |
| | DC 193 Fluid | 0.60 | 3.00 |
| | Boron Nitride | 0.50 | 2.50 |
| | Phenonip | 1.00 | 5.00 |
| | | | |
| **E** | Water | 2.00 | 10.00 |
| | MAP (magnesium ascorbyl phosphate) | 0.05 | 0.25 |
| | | | |
| **F** | Sepigel 305 | 2.20 | 11.00 |

4. The skin lightening composition of claim 1, wherein the composition has the formulation described in the following table
| **Phase** | **Description** | **%** | **Grams** |
|---|---|---|---|
| **A** | Water | 44.85 | 448.50 |
| | TEA (triethanolamine) | 1.70 | 17.00 |
| | Sepiwhite MSH (undecylenoyl phenylalanine) | 0.50 | 5.00 |
| | Propylene Glycol | 6.00 | 60.00 |
| | Glycerine | 2.00 | 20.00 |
| | Disodium EDTA | 0.10 | 1.00 |
| | Prodew 400 | 0.10 | 1.00 |
| | Uninontan U34 (a combination of cucumber extract and lemon extract) | 0.50 | 5.00 |
| | Cosmocair C 250 (creatinine) | 0.20 | 2.00 |
| | Net- DG | 0.10 | 1.00 |
| | AA2G (ascorbic acid 2-glucoside) | 2.00 | 20.00 |
| | Niacinamide | 2.00 | 20.00 |
| | Betafin BP 20 | 1.00 | 10.00 |
| | Actiplex 3691 | 0.50 | 5.00 |
| | Matrixyl 3000 | 2.00 | 20.00 |
| | | | |
| **B** | Carbopol 940 2% | 15.00 | 150.00 |
| | | | |
| **C** | Water | 5.00 | 50.00 |
| | Timiron Super Blue | 1.00 | 10.00 |
| | | | |
| **D** | Permethyl 99A | 4.50 | 45.00 |
| | Dimethicone | 3.50 | 35.00 |
| | Silicone HL-88 | 1.00 | 10.00 |
| | Vitamin E Acetate | 0.10 | 1.00 |
| | DC 193 Fluid | 0.60 | 6.00 |
| | Boron Nitride | 0.50 | 5.00 |
| | Phenonip | 1.00 | 10.00 |
| | | | |
| **E** | Water | 2.00 | 20.00 |
| | MAP (magnesium ascorbyl phosphate) | 0.05 | 0.50 |
| | | | |
| **F** | Sepigel 305 | 2.20 | 22.00 |

5. A non-therapeutic method of lightening skin or evening skin tone, wherein the skin-lightening composition as defined in any of the preceding claims is applied to an age spot, hyperpigmented skin, or a freckle.

6. The composition as defined in any of the preceding claims for use in a method of treating a skin condition wherein the skin condition is hyperpigmented skin, wherein the composition is topically applied to the skin.

## Patentansprüche

1. Hautaufhellende Zusammensetzung, die
(a) Ascorbinsäure-2-Glucosid;
(b) Niacinamid;
(c) eine Kombination aus Gurkenextrakt und Zitronenextrakt;
(d) Creatinin; und
(e) Undecylenoylphenylalanin
umfasst.

2. Hautaufhellende Zusammensetzung nach Anspruch 1, wobei die Zusammensetzung
(a) von 0,01 bis 5 Gew.-% bezogen auf die Zusammensetzung an Ascorbinsäure-2-Glucosid;
(b) von 0,01 bis 5 Gew.-% bezogen auf die Zusammensetzung an Niacinamid;
(c) von 0,01 bis 5 Gew.-% bezogen auf die Zusammensetzung einer Kombination aus Gurkenextrakt und Zitronenextrakt;
(d) von 0,01 bis 5 Gew.-% bezogen auf die Zusammensetzung an Creatinin und
(e) von 0,01 bis 5 Gew.-% bezogen auf die Zusammensetzung an Undecylenoylphenylalanin umfasst.

3. Hautaufhellende Zusammensetzung nach Anspruch 1, wobei die Zusammensetzung die in der nachstehenden Tabelle angegebene Formulierung aufweist
| **Phase** | **Beschreibung** | **%** | **Gramm** |
|---|---|---|---|
| **A** | Wasser | 44,80 | 224,00 |
| | TEA (Triethanolamin) | 1,70 | 8,50 |
| | Sepiwhite MSH (Undecylenoylphenylalanin) | 0,50 | 2,50 |
| | Propylenglycol | 6,00 | 30,00 |
| | Glycerin | 2,00 | 10,00 |
| | Dinatrium EDTA | 0,10 | 0,50 |
| | Prodew 400 | 0,10 | 0,50 |
| | Uninontan U34 (eine Kombination aus Gurkenextrakt und Zitronenextrakt) | 0,50 | 2,50 |
| | Cosmocair C 250 (Creatinin) | 0,20 | 1,00 |
| | Net-DG | 0,10 | 0,50 |
| | AA2G (Ascorbinsäure-2-Glucosid) | 2,00 | 10,00 |
| | Niacinamid | 2,00 | 10,00 |
| | Betafin BP 20 | 1,00 | 5,00 |
| | Actiplex 3535 | 0,50 | 2,50 |
| | Matrixyl 3000 | 2,00 | 10,00 |
| | | | |
| **B** | Carbopol 940 2% | 15,00 | 75,00 |
| | | | |
| **C** | Wasser | 5,00 | 25,00 |
| | Timiron Super Blau | 1,00 | 5,00 |
| | | | |
| **D** | Permethyl 99A | 4,50 | 22,50 |
| | Dimethicon | 3,50 | 17,50 |
| | Silicon HL-88 | 1,00 | 5,00 |
| | Vitamin-E-Acetat | 0,10 | 0,50 |
| | Licorice Ext. | 0,05 | 0,25 |
| | DC 193 Fluid | 0,60 | 3,00 |
| | Bornitrid | 0,50 | 2,50 |
| | Phenonip | 1,00 | 5,00 |
| | | | |
| **E** | Wasser | 2,00 | 10,00 |
| | MAP (Magnesiumascorbylphosphat) | 0,05 | 0,25 |
| | | | |
| **F** | Sepigel 305 | 2,20 | 11,00 |

4. Hautaufhellende Zusammensetzung nach Anspruch 1, wobei die Zusammensetzung die in der nachstehenden Tabelle angegebene Formulierung aufweist
| **Phase** | **Beschreibung** | **%** | **Gramm** |
|---|---|---|---|
| **A** | Wasser | 44,85 | 448,50 |
| | TEA (Triethanolamin) | 1,70 | 17,00 |
| | Sepiwhite MSH (Undecylenoylphenylalanin) | 0,50 | 5,00 |
| | Propylenglycol | 6,00 | 60,00 |
| | Glycerin | 2,00 | 20,00 |
| | Dinatrium EDTA | 0,10 | 1,00 |
| | Prodew 400 | 0,10 | 1,00 |
| | Uninontan U34 (eine Kombination aus Gurkenextrakt und Zitronenextrakt) | 0,50 | 5,00 |
| | Cosmocair C 250 (Creatinin) | 0,20 | 2,00 |
| | Net-DG | 0,10 | 1,00 |
| | AA2G (Ascorbinsäure-2-Glucosid) | 2,00 | 20,00 |
| | Niacinamid | 2,00 | 20,00 |
| | Betafin BP 20 | 1,00 | 10,00 |
| | Actiplex 3691 | 0,50 | 5,00 |
| | Matrixyl 3000 | 2,00 | 20,00 |
| | | | |
| **B** | Carbopol 940 2% | 15,00 | 150,00 |
| | | | |
| **C** | Wasser | 5,00 | 50,00 |
| | Timiron Super Blau | 1,00 | 10,00 |
| | | | |
| **D** | Permethyl 99A | 4,50 | 45,00 |
| | Dimethicon | 3,50 | 35,00 |
| | Silicon HL-88 | 1,00 | 10,00 |
| | Vitamin-E-Acetat | 0,10 | 1,00 |
| | DC 193 Fluid | 0,60 | 6,00 |
| | Bornitrid | 0,50 | 5,00 |
| | Phenonip | 1,00 | 10,00 |
| | | | |
| **E** | Wasser | 2,00 | 20,00 |
| | MAP (Magnesiumascorbylphosphat) | 0,05 | 0,50 |
| | | | |
| **F** | Sepigel 305 | 2,20 | 22,00 |

5. Ein nicht-therapeutisches Verfahren zum Aufhellen von Haut oder zum Ausgleichen der Hauttönung, wobei die hautaufhellende Zusammensetzung gemäß einem der vorangehenden Ansprüche auf einen Altersfleck, über pigmentierte Haut oder Sommersprossen aufgetragen wird.

6. Zusammensetzung nach einem der vorhergehenden Ansprüche zur Verwendung in einem Verfahren zur Behandlung eines Hautzustands, wobei der Hautzustand überpigmentierte Haut ist und wobei die Zusammensetzung topisch auf die Haut aufgebracht wird.

## Revendications

1. Composition de blanchiment de la peau comprenant :
(a) de l'acide 2-glucoside ascorbique ;
(b) du niacinamide ;
(c) une combinaison d'extrait de concombre et d'extrait de citron ;
(d) de la créatinine; et
(e) de la undécylènoyl phénylalanine.

2. Composition de blanchiment de la peau selon la revendication 1, laquelle composition comprend :
(a) de 0,01 % à 5 % en poids de la composition d'acide 2-glucoside ascorbique;
(b) de 0,01 % à 5 % en poids de la composition de niacinamide;
(c) de 0,01 % à 5 % en poids de la composition d'une combinaison d'extrait de concombre et d'extrait de citron;
(d) de 0,01 % à 5 % en poids de la composition de créatinine; et
(e) de 0,01 % à 5 % en poids de la composition d'undécylènoyl phénylalanine.

3. Composition de blanchiment de la peau selon la revendication 1, laquelle composition possède la formulation décrite dans le tableau suivant
| Phase | Description | % | Grammes |
|---|---|---|---|
| A | Eau | 44,80 | 224,00 |
| | TEA (triéthanolamine) | 1,70 | 8,50 |
| | Sepiwhite MSH (undécylènoyl phénylalanine) | 0,50 | 2,50 |
| | Propylène Glycol | 6,00 | 30,00 |
| | Glycérine | 2,00 | 10,00 |
| | EDTA disodique | 0,10 | 0,50 |
| | Prodew 400 | 0,10 | 0,50 |
| | Uninontan U34 (une combinaison d'extrait de concombre et d'extrait de citron) | 0,50 | 2,50 |
| | Cosmocair C 250 (créatinine) | 0,20 | 1,00 |
| | Net- DG | 0,10 | 0,50 |
| | AA2G (acide 2-glucoside ascorbique) | 2,00 | 10,00 |
| | Niacinamide | 2,00 | 10,00 |
| | Betafin BP 20 | 1,00 | 5,00 |
| | Actiplex 3535 | 0,50 | 2,50 |
| | Matrixyl 3000 | 2,00 | 10,00 |
| | | | |
| B | Carbopol 940 2% | 15,00 | 75,00 |
| | | | |
| C | Eau | 5,00 | 25,00 |
| | Timiron Super Blue | 1,00 | 5,00 |
| | | | |
| D | Perméthyl 99A | 4,50 | 22,50 |
| | Diméthicone | 3,50 | 17,50 |
| | Silicone HL-88 | 1,00 | 5,00 |
| | Vitamine E Acétate | 0,10 | 0,50 |
| | Extrait de réglisse | 0,05 | 0,25 |
| | DC 193 Fluide | 0,60 | 3,00 |
| | Nitrure de bore | 0,50 | 2,50 |
| | Phénonip | 1,00 | 5,00 |
| | | | |
| E | Eau | 2,00 | 10,00 |
| | MAP (magnésium ascorbyl phosphate) | 0,05 | 0,25 |
| | | | |
| F | Sepigel 305 | 2,20 | 11,00 |

4. Composition de blanchiment de la peau selon la revendication 1, laquelle composition possède la formulation décrite dans le tableau suivant
| Phase | Description | % | Grammes |
|---|---|---|---|
| A | Eau | 44,85 | 448,50 |
| | TEA (triéthanolamine) | 1,70 | 17,00 |
| | Sepiwhite MSH (undécylènoyl phénylalanine) | 0,50 | 5,00 |
| | Propylène Glycol | 6,00 | 60,00 |
| | Glycérine | 2,00 | 20,00 |
| | EDTA disodique | 0,10 | 1,00 |
| | Prodew 400 | 0,10 | 1,00 |
| | Uninontan U34 (une combinaison d'extrait de concombre et d'extrait de citron) | 0,50 | 5,00 |
| | Cosmocair C 250 (créatinine) | 0,20 | 2,00 |
| | Net- DG | 0,10 | 1,00 |
| | AA2G (acide 2-glucoside ascorbique) | 2,00 | 20,00 |
| | Niacinamide | 2,00 | 20,00 |
| | Betafin BP 20 | 1,00 | 10,00 |
| | Actiplex 3691 | 0,50 | 5,00 |
| | Matrixyl 3000 | 2,00 | 20,00 |
| | | | |
| B | Carbopol 940 2% | 15,00 | 150,00 |
| | | | |
| C | Eau | 5,00 | 50,00 |
| | Timiron Super Blue | 1,00 | 10,00 |
| | | | |
| D | Perméthyl 99A " | 4,50 | 45,00 |
| | Diméthicone | 3,50 | 35,00 |
| | Silicone HL-88 | 1,00 | 10,00 |
| | Vitamine E Acétate | 0,10 | 1,00 |
| | DC 193 Fluide | 0,60 | 6,00 |
| | Nitrure de bore | 0,50 | 5,00 |
| | Phénonip | 1,00 | 10,00 |
| | | | |
| E | Eau | 2,00 | 20,00 |
| | MAP (magnésium ascorbyl phosphate) | 0,05 | 0,50 |
| | | | |
| F | Sepigel 305 | 2,20 | 22,00 |

5. Procédé non thérapeutique de blanchiment de la peau ou d'homogénéisation du teint de peau, dans lequel la composition de blanchiment de la peau telle que définie dans l'une quelconque des revendications précédentes est appliquée à une tache de vieillissement, une peaux hyperpigmentée ou une tache de rousseur.

6. Composition telle que définie dans l'une quelconque des revendications précédentes destinée à une utilisation dans un procédé de traitement d'un état de la peau où l'état de la peau est une peau hyperpigmentée, où la composition est appliquée de manière topique à la peau.
